# EUROPÄISCHE PATENTSCHRIFT

(11) **EP 2 155 871 B1**
(45) Veröffentlichungstag und Bekanntmachung des Hinweises auf die Patenterteilung: **12.08.2015**
(21) Anmeldenummer: 08756821.8
(22) Anmeldetag: 12.06.2008
(51) Int. Cl.: C12N 9/64, A61K 38/48

(54) **ACE2 POLYPEPTID**
ACE2 POLYPEPTIDE
POLYPEPTIDE ACE2

(30) Priorität: 12.06.2007 AT 9132007; 08.04.2008 EP 08450052
(43) Veröffentlichungstag der Anmeldung: 24.02.2010
(60) Teilanmeldung: 12165189.7
(73) Patentinhaber: Apeiron Biologics AG, 1030 Wien (AT)
(72) Erfinder: SCHUSTER, Manfred, A-2191 Schrick (AT); LOIBNER, Hans, A-1230 Wien (AT); JANZEK-HAWLAT, Evelyne, A-1230 Wien (AT); PEBALL, Bernhard, A-1070 Wien (AT); STRANNER, Stefan, A-1230 Wien (AT); WAGNER, Bettina, A-2700 Wiener Neustadt (AT); WEIK, Robert, A-2724 Hohe Wand-Stollhof (AT)
(74) Vertreter: Sonn & Partner Patentanwälte
(86) Internationale Anmeldenummer: PCT/AT2008/000211
(87) Internationale Veröffentlichungsnummer: WO 2008/151347

(56) Entgegenhaltungen:
- EP-A1- 2 108 695
- VINCENT M J ET AL: "Chloroquine is a potent inhibitor of SARS coronavirus infection and spread" VIROLOGY JOURNAL 20050822 GB, Bd. 2, 22. August 2005 (2005-08-22), XP021010915 ISSN: 1743-422X 1743-422X
- WARNER FIONA J ET AL: "Angiotensin-converting enzyme 2 (ACE2), but not ACE, is preferentially localized to the apical surface of polarized kidney cells." THE JOURNAL OF BIOLOGICAL CHEMISTRY 25 NOV 2005, Bd. 280, Nr. 47, 25. November 2005 (2005-11-25), Seiten 39353-39362, XP002490917 ISSN: 0021-9258
- PEPTIDE AND PROTEIN-BASED THERAPEUTICS Pioneering New Frontiers January 7-9, 2008 - Hotel Del Coronado - San Diego, California Downloaded 30.07.2008 from http://www.infoshop-japan.com/conference/p eptalk/pdf/peptide-proteinbased.pdf page 11, Development of an ACE2 Enzyme Substitution Therapy XP002490920
- KOST O A ET AL: "New feature of angiotensin-converting enzyme: carbohydrate-recognizing domain." JOURNAL OF MOLECULAR RECOGNITION : JMR 2000 NOV-DEC, Bd. 13, Nr. 6, November 2000 (2000-11), Seiten 360-369, XP002490918 ISSN: 0952-3499
- PRABAKARAN P ET AL: "A model of the ACE2 structure and function as a SARS-CoV receptor" BIOCHEMICAL AND BIOPHYSICAL RESEARCH COMMUNICATIONS, ACADEMIC PRESS INC. ORLANDO, FL, US, Bd. 314, Nr. 1, 30. Januar 2004 (2004-01-30), Seiten 235-241, XP004483586 ISSN: 0006-291X
- VICKERS CHAD ET AL: "Hydrolysis of biological peptides by human angiotensin-converting enzyme-related carboxypeptidase" JOURNAL OF BIOLOGICAL CHEMISTRY, AMERICAN SOCIETY OF BIOLOCHEMICAL BIOLOGISTS, BIRMINGHAM,; US, Bd. 277, Nr. 17, 26. April 2002 (2002-04-26), Seiten 14838-14843, XP002464545 ISSN: 0021-9258 in der Anmeldung erwähnt
- IMAI Y ET AL: "Angiotensin-converting enzyme 2 protects from severe acute lung failure" NATURE, NATURE PUBLISHING GROUP, LONDON, UK, Bd. 436, Nr. 7047, 7. Juli 2005 (2005-07-07), Seiten 112-116, XP002349987 ISSN: 0028-0836
- TIPNIS ET AL: "A human homolog of Angiotensin-converting enzyme" JOURNAL OF BIOLOGICAL CHEMISTRY, AMERICAN SOCIETY OF BIOLOCHEMICAL BIOLOGISTS, BIRMINGHAM,; US, Bd. 275, Nr. 43, 27. Oktober 2000 (2000-10-27), Seiten 33238-33243, XP002207551 ISSN: 0021-9258
- GUY J L ET AL: "Membrane-associated zinc peptidase families: comparing ACE and ACE2" BIOCHIMICA ET BIOPHYSICA ACTA (BBA) - PROTEINS & PROTEOMICS, ELSEVIER, Bd. 1751, Nr. 1, 1. August 2005 (2005-08-01), Seiten 2-8, XP004995171 ISSN: 1570-9639
- DANIEL R. BENIAC ET AL: "Conformational Reorganization of the SARS Coronavirus Spike Following Receptor Binding: Implications for Membrane Fusion", PLOS ONE, vol. 2, no. 10, 1 January 2007 (2007-01-01), pages E1082-E1082, XP55025522, ISSN: 1932-6203, DOI: 10.1371/journal.pone.0001082
- Stryer: "Biochemistry, 3rd Ed", page 893, * page 893 *
- "Material data sheet of Adipongen", , 1 January 2010 (2010-01-01),
- Voet, Voet: "Biochemistry 1995, 2nd edition", pages 332-337,
- Pschyrembel: "Klinisches Wörterbuch, Auflage 256", pages 358-359,

## Beschreibung

Die vorliegende Erfindung betrifft das Gebiet der Produktion rekombinanter Proteine.

Angiotensin Converting Enzyme (ACE2) stellt ein Schlüsselenzym des Renin-Angiotensin-Systems dar. Als Carboxypeptidase wird es membranverankert, als Rezeptor vor allem auf Lungen-, Nieren- und Herzzellen, aber auch auf Endothelzellen exprimiert und spaltet diverse Peptidsubstrate. Prominente Substratvertreter sind Angiotensin II (Ang II), welches zu Angiotensin 1-7 (Ang 1-7) gespalten wird, Angiotensin-I, welches zu Angiotensin 1-9 gespalten wird, aber ebenso Apelin und Bradykinin. Ang II und Ang 1-7 sind Antagonisten des Renin-Angiotensin-Systems. ACE2 ist über die Steuerung der Peptidverhältnisse maßgeblich für die Regulation der Gefäßdicke sowie für die Endothelpermeabilität verantwortlich und beeinflusst hierbei die Homeostase des Organismus. Die Expression von ACE2 ist u.a. zytokingesteuert und wird in diversen entzündlichen Erkrankungen herabgesetzt, was in weiterer Folge zu einer pathologischen Anreicherung von Ang II, einem der wichtigsten Substrate von ACE2, führt.

ACE2 dient zur Behandlung von ARDS oder ALI, zwei Formen einer akuten Lungenerkrankung, mit welchen eine herunterregulierte ACE2 Expression in der Lunge einhergeht. Für diese Therapie wird rekombinantes lösliches humanes ACE2 verwendet, welches systemisch gegeben wird und binnen kürzester Zeit im gesamten Organismus zur Verfügung steht, um eine erhöhte Ang II Konzentration abzubauen und dabei Ang 1-7 zu erzeugen. Dies kompensiert die negativen Auswirkungen erhöhter Ang II Konzentrationen. Hierfür ist es wünschenswert ein Produkt in Händen zu haben, das ein geeignetes pharmakologisches Profil aufweist: Dementsprechend kennzeichnet sich eine geeignete Substanz durch eine gute Verteilung im Organismus, eine pharmakologisch sinnvolle Halbwertszeit sowie durch eine geringe Immunogenizität. Insbesondere muss das Produkt enzymatisch aktiv, gut löslich sowie auch in Lösung stabil sein und sich in hoher Reinheit reproduzierbar und ökonomisch darstellen lassen.

Tipnis et al. (J Biol Chem. 275 (43) (2000): 33238-43) beschreibt die Isolierung von ACE2 (hier noch als ACEH bezeichnet) und seine cDNA-Isolierung. Durch Produktion in CHO-Zellen wurde ein glykosyliertes Protein-Monomer mit einer Molmasse von 120 kDa erhalten. Nach Deglykoslierung wies dieses Protein eine Masse von 85 kDa auf.

Donoghue (Circ Res. 87 (5) (2000): 1-9) beschreibt die Expression von löslichem ACE2 in CHO-Zellen, welches nicht voll glykosyliert ist und mit einer Molmasse von ca. 90 kDa charakterisiert wurde. Dieses Dokument betrifft des Weiteren Sequenzvergleiche diverser ACE2-Sequenzen und Anti-ACE2-Antikörper.

WO 2004/023270 A2 betrifft die Kristallisierung von ACE2 nach der Expression von ACE2-Monomeren in Sf9 Insektenzellen. Die Molmasse des Proteins wurde dabei mit 89,6 kDa nach einer Massenspektrometrieanalyse angegeben.

Vincent et al. (Virology Journal 2 (69)2005: doi: 10.1186/1743-422X-2-69) betriff einen pharmazeutischen Wirkstoff, Chloroquin, welcher in die terminale Glykosilierung von ACE2 eingreift. Durch veränderte Glykosilierung ist ein gebundenes ACE2 kein bzw. ein schlechterer Rezeptor für das SARS-CoV.

Warner et al. (J. Biol. Chem. 280 (47) 2005: 39353-39362) beschreibt die rekombinante Expression von ACE2 und ACE in CHO-Zellen und MDCKII-Zellen. In CHO-Zellen exprimiertes ACE2 wurde als ein einzelnes ~120 kDa Polypeptid in gelösten Membranpräparationen durch Western-blotting detektiert. In MDCKII-Zellen exprimiertes ACE2, welches als komplett glykosyliert beschrieben wird, wurde als einzelne ~120 kDa Proteinbande, welche durch Deglykosylierung nach ~92 kDa gewandert ist, in Western-blotting beschrieben.

Manfred Schuster, "Peptide and protein-based therapeutics" (Mining The Plasma Proteome, Hotel Del Coronado (2008): 1-11) ist ein Abstract-Heft des Cambridge Healthtech Institutes zu einer Konferenz, in der die Produktion von ACE2 in CHO-Zellen vorgestellt wurde.

Kost et al. (J. Mol. Recognit. 13 (2000):360-369) zeigt Untersuchungen zur Dimerisierung von ACE.

Prabakaran et al. (Biochem. Biophys. Res. Com. 314, (2004): 235-241) beschreibt eine 3D Struktur von ACE2 und deren Relevanz als Rezeptor für das SARS-CoV.

Vickers et al. (J. Biol. Chem. 277 (2002):14838-14843) beschreibt Eigenschaften von ACE2 welches in Insekten SF9-Zellen hergestellt wurde.

Imai et al. (Nature 436 (2005): 112-116) betrifft die Behandlung von ARDS mittels ACE2.

Tipnis et al. (J. Biol. Chem. 275 (2000): 33238-33243) und Guy et al. (Biochimica et Biophysica Acta 1751 (2005): 2-8) betreffen Studien zu Unterschieden zwischen ACE2 (auch. als ACEH bezeichnet) und ACE.

Die therapeutische Umsetzung einer effizienten Enzymsubstitutionstherapie erfordert einen Herstellungsprozess, der ökonomisch und reproduzierbar ein hochreines pharmakologisch effizientes Produkt darstellt. Der lösliche, extrazelluläre Abschnitt von ACE2 enthält 7 N-Glykosylierungsstellen. Nicht-glykosyliertes ACE2 ist schlecht löslich, neigt zur Aggregation, ist vermehrt immunogen und weist eine verkürzte Halbwertszeit auf. Außerdem besitzt es einen kleineren hydrodynamischen Durchmesser, welcher vor allem die Aufreinigung negativ beeinflusst. Es ist daher ein Ziel der vorliegenden Erfindung hoch aktives ACE2 mit einer guten in vivo Halbwertszeit zur Verfügung zu stellen. Dieses Ziel wird durch die Gegenstände der Ansprüche erfüllt.

Die vorliegende Erfindung betrifft rekombinante, glykosylsierte, lösliche, menschliche, dimere, enzymatisch aktive ACE2 Polypeptide. Das erfindungsgemäße ACE2 Polypeptid ist glykosyliert, wobei vorzugsweise die Glycogruppen eines ACE2 Polypeptid Monomers in Summe mindestens 10, 11, 12, 13, 14, 15, oder mindestens 16 Sialylsäurereste, vorzugsweise mindestestens 14 Sialylsäurereste, aufweisen und wobei das ACE2 Polypeptid als Dimer vorliegt. Ebenso ist hierin ein ACE2 Monomer mit den hierin genannten Glykosylierungen, Molgewichten und weiteren Spezifizierungen beschrieben. Das Dimer ist vorzugsweise ein Homo-Dimer, seine Monomereinheiten haben im Speziellen eine identische Sequenz und Glycosylierung. In der Regel sind die Monomereinheiten des Dimers nicht-kovalent verbunden. Ein Monomer kann aus dem Dimer durch oder denaturierende Schritte ohne Weiteres erhalten werden. Alle hierhin genannten vorzugsweise Glycosylierungen betreffen sowohl das Dimer als auch das Monomer, inklusive eines oder beider Monomere des Dimerkomplexes. Insbesondere bevorzugt enthält das Dimer zwei Zink Ionen.

Unter Sialylsäurereste werden insbesondere Reste vom N-Acetylneuraminsäure-Typ (Neu5Ac) verstanden, im speziellen an N- oder O-Glycosylierungen.

Grundsätzlich ist die Stabilität eines Therapeutikums ein sehr wichtiges Kriterium für dessen Halbwertszeit und somit dessen Wirksamkeit.

Rekombinantes humanes ACE2 wurde bisher ausschließlich als Monomer identifiziert und ist auch als solches in der Literatur hinsichtlich seiner Funktionalität, seiner Kristallstruktur sowie hinsichtlich seiner Interaktion mit einem hochspezifischen Inhibitor beschrieben.

So beschreibt beispielsweise Towler et al. (J Biol Chem 279(17), 2004: 17996-18007) unter Verweis auf Vickers et al. (J Biol Chem 277(17), 2002: 14838-14843) die Expression von ACE2 in Sf9 Insektenzellen, welche nach schonender Größenausschlusschromatographie als letzten Schritt der Aufreinigung als Monomere erhalten wurden. Die Molmasse eines Monomers betrug 89,6 kDa.

Käuflich erwerbbares ACE2 liegt ebenfalls in Monomerform vor (von R&D Systems, Katalognummer 933-ZN, Lot Nummer FIU035071) welches nach dem in Tipnis et al. (2000, oben) genannten Verfahren hergestellt wird, mit dem Unterschied, dass als Expressionssystem NS0 anstelle von CHO Zellen verwendet werden. Es werden Monomere beschrieben, die sowohl unter reduzierenden und nichtreduzierenden Bedingungen ein Molgewicht von 120 kDa aufweisen.

Gemäß Donoghue et al. (Circ Res 87, 2000: e1-e9) wurden ACE2 Monomere in CHO Zellen exprimiert, die nach Sezernierung ein Molekulargewicht von 90 kDa zeigten.

Alle hierin angeführten Dokumente und Veröffentlichungen sind durch Bezugnahme hierin aufgenommen.

Zur Stabilisierung des erfindungsgemäßen Therapeutikums wurde ein Verfahren entwickelt, welches ausschließlich zu stabilen ACE2 Dimeren führt, die sowohl produktionstechnische als auch pharmakologische Vorteile im Vergleich zu ACE2 Monomeren aufweisen.

Die Dimerisierung erbringt die folgenden Vorteile:
- Bessere Löslichkeit und Bio-Verfügbarkeit in physiologischen Lösungen: Das Dimer zeigt eine hohe Löslichkeit und längere in vivo Halbwertszeit und Verfügbarkeit.
- Keine Aggregatbildung: Das ACE2 Dimer bildet stabile Komplexe ohne Anlagerung weiterer ACE2 Moleküle zum Dimer.
-
- Reduzierter Proteaseangriff: Nachdem einzelne Proteinabschnitte und hierbei aller Wahrscheinlichkeit nach der C-terminale Teil in das Innere des Dimers gerichtet sind, wird dieser C-Terminus nicht abgebaut.
- Gesteigerte Halbwertszeit: Durch die geringere Immunogenität, die bessere Löslichkeit und der reduzierten Anfälligkeit für proteolytischen Abbau wird die Halbwertszeit des Proteins erhöht.
- Einfachere Proteinreinigung: Der hydrodynamische Durchmesser des ACE2 Dimers ist aufgrund seiner Struktur und einer ausgeprägten Solvatisierungshülle größer als der errechnete. Deswegen lassen sich ACE2 Dimere hervorragend von klassischen Verunreinigungen aus der Proteinexpression, wie zum Beispiel Serum Albumin (67 kDa) durch Größehtrennungssäulen abtrennen.

Vorzugsweise ist das rekombinante ACE2 Polypeptid an mindestens 80% der möglichen N-Glykosylierungspositonen glykosyliert und weist einen Zuckeranteil von größer als 10% (Massen-% des gesamten ACE2) oder 11%, 12%, 13%, 14%, vorzugsweise größer als 15% oder 16%, 17%, 18%, 19%, insbesondere größer als 20 % oder 21%, 22%, 23% 24% oder 25%, auf.

Es wurde ein Produktionsprozess entwickelt, der hochreines und enzymatisch aktives, stark komplex glykosyliertes ACE2 reproduzierbar darstellt. Das Produkt zeichnet sich durch seinen hohen Zuckeranteil (>20 Massen %) und die komplexen, stark verzweigten, teilweise negativ geladenen Zuckerstrukturen aus. Diese wirken sich positiv auf die Löslichkeit, die Bioverfügbarkeit, die enzymatische Aktivität sowie die pharmakologischen Eigenschaften des Produktes aus. Durch die Wahl eines geeigneten Expressionskonstruktes, eines geeigneten Expressionswirtes, einer optimierten Selektionsstrategie, durch ein auf den Zellmetabolismus abgestimmtes Medium sowie durch minutiöse begleitende Klonanalytik und Selektion konnte eine Zelllinie hergestellt werden, die das gewünschte Produkt sezerniert.

ACE2 wurde bereits in Insektenzellen Sf9 und Mauszellen NS0 exprimiert. Das Material wurde vor allem in in vitro Versuchen eingesetzt. Es gibt auch Resultate über transiente Expression von ACE2 in CHO Zellen. Bis jetzt wurde noch keine Zelllinie mit prozesstauglicher Produktivität hergestellt. Weiters wurde noch keine dementsprechende Klonselektion hinsichtlich der Produkteigenschaften, im Speziellen in Bezug auf die N-Glykosylierung, durchgeführt.

Die Löslichkeit eines Proteins wird nicht nur durch seine Aminosäuresequenz, sondern auch durch seine Faltung sowie durch post-translationale Modifikationen bestimmt. Es sind vor allem geladene Zuckerstrukturen, die maßgeblich die Löslichkeit eines Proteins steigern und dessen pharmakologisches Profil beeinflussen. So konnte für EPO aufgezeigt werden, dass die Anwesenheit von komplexen verzweigten Glykostrukturen die Halbwertszeit dieses Proteins positiv beinflusst.

Prinzipiell kommen zur rekombinanten Expression von ACE2 verschiedene Expressionssysteme in Frage, wobei prokaryontische Wirtszellen aufgrund fehlender Prozessierung von N-Glykosylierungsstellen nicht weiter getestet wurden.

Vorzugsweise weisen mindestens 70% der glykosylierten N-Glykosylierungspositionen eine Struktur unabhängig voneinander ausgewählt aus den Formeln 1-8: 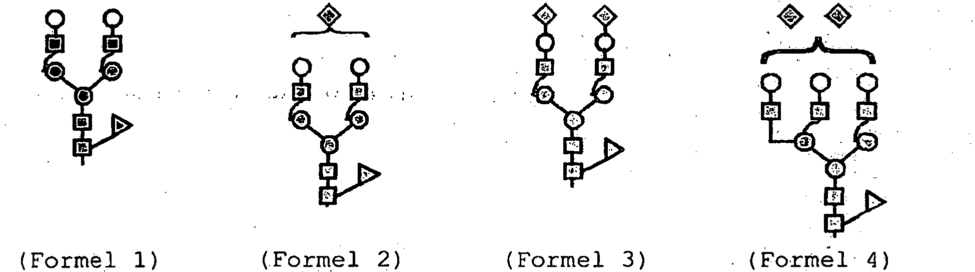 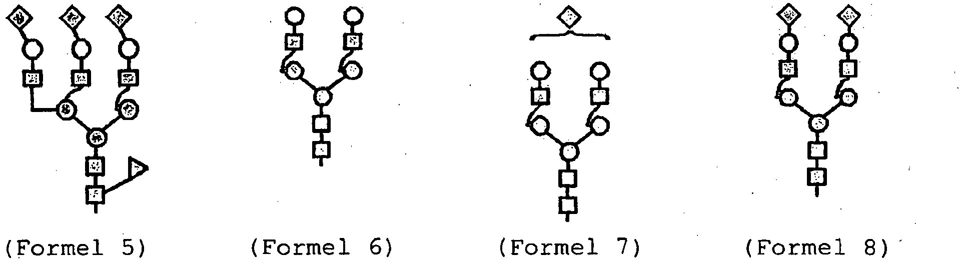 wobei

| | | | |
|---|---|---|---|
| G lc N A c | 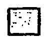 | F u c | 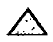 |
| M a n | 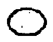 | X y l | 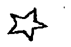 |
| G a l | 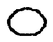 | N e u 5 A c | 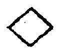 |

ist, auf.

Vorzugsweise sind alle der möglichen N-Glykosylierungspositionen glykosyliert.

Vorzugsweise weisen mindestens 80%, vorzugsweise mindestens 90%, insbesondere 100%, der glykosylierten N-Glykosylierungspositionen eine Struktur der Formeln 1-8 auf.

Vorzugsweise hat ein ACE2 Monomereinheit des Dimers ein Molekulargewicht von mindestens 90 kDa, vorzugsweise mindestens 92 kDa, besonders bevorzugt mindestens 94 kDa, insbesondere mindestens 96 kDa, speziell bevorzugt mindestens 98 kDa, am meisten bevorzugt mindestens 100 kDa, 100,5 kDa, 101 kDa, 101,5 kDa oder auch mindestens 102 kDa. Eine absolute Molmasse - also des Peptids an sich ohne Hydrathülle - kann durch Peptide-map bestimmt werden. Höher glykosylierte Formen können auch Molmassen von mindestens 103 kDa, 104 kDa, 105 kDa, 106 kDa, 107 kDa oder mindestens 108 kDa aufweisen.

Molmassebestimmungen, welche durch weitere Faktoren, wie Hydrathüllen beeinflusst werden - wie z.B. Chromatographien oder Gelelektrophoresen in wässrigen Systemen - können auch zu höheren Ergebnissen führen. In weiteren Ausführungsformen hat eine ACE2 Monomereinheit des Dimers ein scheinbares Molekulargewicht von mindestens 101 kDa oder mindestens 102 kDa, vorzugsweise mindestens 105 kDa, besonders bevorzugt mindestens 109 kDa, insbesondere mindestens 113 kDa, speziell bevorzugt mindestens 177 kDa, am meisten bevorzugt mindestens 119 kDa, wie durch Gelelektrophorese bestimmt wird.

In anderen Ausführungsformen ist das Molekulargewicht des Monomers (scheinbar oder absolut) maximal 102 kDa, 103 kDa, 104 kDa, 108 kDa, 110 kDa, 112 kDa, 116 kDa, 120 kDa, 125 kDa, 130 kDa, 135 kDa oder 140 kDa. Höhere Molekulargewichte sind durch Modifizierungen von ACE2 möglich, z.B. PEGylierung.

Vorzugsweise hat das Monomer (welches kein Dimer bildet) ein Molekulargewicht von mindestens 82 kDa, vorzugsweise mindestens 86 kDa, besonders bevorzugt mindestens 90 kDa, insbesondere mindestens 94 kDa, speziell bevorzugt mindestens 98 kDa, am meisten bevorzugt mindestens 101 kDa bzw. maximal 102 kDa, 103 kDa, 104 kDa, 108 kDa, 110 kDa oder maximal 112 kDa. Diese Molekulargewichte können z.B. nach der Peptide-map-Methode festgestellt werden.

Vorzugsweise weist das ACE2 Polypeptid keine transmembrane Domäne auf. Es handelt sich daher um lösliches ACE2. Besonders bevorzugte Ausführungsformen umfassen dabei lösliche ACE2-Polypeptide, deren Polypeptidkette aus den Aminosäuren 18-740 oder enzymatisch aktiven Fragmenten davon bestehen. Ein weiteres Polypeptid besteht aus den Aminosäuren 18-615 der SEQ ID NO: 1.

Obwohl menschliches ACE2 (SEQ ID NO:1) für die meisten therapeutischen Verwendungen bevorzugt ist, kann auch ACE2 von anderen Säugetieren, z.B. Maus, Ratte, Hamster, Schwein, Primaten oder Rind verwendet werden. ACE2 ist ein universelles Enzym in allen Säugetieren mit dem in verschiedenen Spezies identischen Substrat Ang II. Daher ist es grundsätzlich auch in Fremdorganismen einsetzbar. Somit kann ACE2 Protein unabhängig vom Ursprung von ACE2 z.B. Menschen, Mäuse, Ratten, Hamster, Schweine, Primaten oder Rinder behandelt werden. Vorzugsweise ist jedoch der Ursprung von ACE2 und des behandelten Organismus gleich.

Vorzugsweise ist ein zu Ser740 der SEQ ID NO: 1 (z.B. der C-Terminus) korrespondierendes Serin (oder C-terminale Aminosäure) des ACE2 Polypeptids O-glykosyliert.

Vorzugsweise weisen mind. 70%, vorzugsweise mind. 80%, insbesondere mind. 90%, am meisten bevorzugt 100% der glykosyliertten N-Glykosylierungsstellen Sialinsäure auf, vorzugsweise sind die N-Glykosylierungsstellen entsprechend Asn53, Asn90, Asn103, Asn322, Asn432, Asn546, Asn690 der SEQ ID NO:1 sialysiert.

In speziellen Ausführungen ist ein zu Asn53 der SEQ ID NO: 1 korrespondierendes Asparagin einfach, zweifach, dreifach oder vierfach sialylisiert. In einer ACE2 Präparation sind vorzugsweise mindestens 50%, 60%, 70%, 80%, 90%, 95%, 99% oder 100% dieser Aminosäure entweder einfach, zweifach, dreifach oder vierfach sialylisiert.

In speziellen Ausführungen ist ein zu Asn90 der SEQ ID NO: 1 korrespondierendes Asparagin einfach, zweifach, dreifach oder vierfach sialylisiert. In einer ACE2 Präparation sind vorzugsweise mindestens 50%, 60%, 70%, 80%, 90%, 95%, 99% oder 100% dieser Aminosäure entweder einfach, zweifach, dreifach oder vierfach sialylisiert.

In speziellen Ausführungen ist ein zu Asn103 der SEQ ID NO: 1 korrespondierendes Asparagin einfach, zweifach, dreifach oder vierfach sialylisiert. In einer ACE2 Präparation sind vorzugsweise mindestens 50%, 60%, 70%, 80%, 90%, 95%, 99% oder 100% dieser Aminosäure entweder einfach, zweifach, dreifach oder vierfach sialylisiert.

In speziellen Ausführungen ist ein zu Asn322 der SEQ ID NO: 1 korrespondierendes Asparagin einfach, zweifach, dreifach oder vierfach sialylisiert. In einer ACE2 Präparation sind vorzugsweise mindestens 50%, 60%, 70%, 80%, 90%, 95%, 99% oder 100% dieser Aminosäure entweder einfach, zweifach, dreifach oder vierfach sialylisiert.

In speziellen Ausführungen ist ein zu Asn432 der SEQ ID NO: 1 korrespondierendes Asparagin einfach, zweifach, dreifach oder vierfach sialylisiert. In einer ACE2 Präparation sind vorzugsweise mindestens 50%, 60%, 70%, 80%, 90%, 95%, 99% oder 100% dieser Aminosäure entweder einfach, zweifach, dreifach oder vierfach sialylisiert.

In speziellen Ausführungen ist ein zu Asn546 der SEQ ID NO: 1 korrespondierendes Asparagin einfach, zweifach, dreifach oder vierfach sialylisiert. In einer ACE2 Präparation sind vorzugsweise mindestens 50%, 60%, 70%, 80%, 90%, 95%, 99% oder 100% dieser Aminosäure entweder einfach, zweifach, dreifach oder vierfach sialylisiert.

In speziellen Ausführungen ist ein zu Asn690 der SEQ ID NO: 1 korrespondierendes Asparagin einfach, zweifach, dreifach oder vierfach sialylisiert. In einer ACE2 Präparation sind vorzugsweise mindestens 50%, 60%, 70%, 80%, 90%, 95%, 99% oder 100% dieser Aminosäure entweder einfach, zweifach, dreifach oder vierfach sialylisiert.

In speziellen Ausführungen ist ein zu Ser740 der SEQ ID NO: 1 korrespondierendes Serin einfach, zweifach, dreifach oder vierfach sialylisiert. In einer ACE2 Präparation sind vorzugsweise mindestens 50%, 60%, 70%, 80%, 90%, 95%, 99% oder 100% dieser Aminosäure entweder einfach, zweifach, dreifach oder vierfach sialylisiert.

Vorzugsweise weisen mind. 30%, vorzugsweise mind. 40%, insbesondere mind. 55%, am meisten bevorzugt mind. 70% der glykosylierten N-Glykosylierungsstellen mindestens zwei Sialinsäuren auf. In einer ACE2 Präparation sind vorzugsweise mindestens 50%, 60%, 70%, 80%, 90%, 95%, 99% oder 100% dieser Aminosäure derart sialylisiert.

Vorzugsweise ist ein zu Asn53 der SEQ ID NO: 1 korrespondierendes Asparagin N-glykosyliert, vorzugsweise mit einem Glykan der Strukturen nach Formel 1, 2, 3, 4, 5, 6, 7 oder 8.

Vorzugsweise ist ein zu, Asn90 der SEQ ID NO: 1 korrespondierendes Asparagin N-glykosyliert, vorzugsweise mit einem Glykan der Strukturen nach Formel 1, 2, 3, 4, 5, 6, 7 oder 8.

Vorzugsweise ist ein zu Asn103 der SEQ ID NO: 1 korrespondierendes Asparagin N-glykosyliert, vorzugsweise mit einem Glykan der Strukturen nach Formel 1, 2, 3, 4, 5, 6, 7 oder 8.

Vorzugsweise ist ein zu Asn322 der SEQ ID NO: 1 korrespondierendes Asparagin N-glykosyliert, vorzugsweise mit einem Glykan der Strukturen nach Formel 1, 2, 3, 4, 5, 6, 7 oder 8.

Vorzugsweise ist ein zu Asn432 der SEQ ID NO: 1 korrespondierendes Asparagin N-glykosyliert, vorzugsweise mit einem Glykan der Strukturen nach Formel 1, 2, 3, 4, 5, 6, 7 oder 8.

Vorzugsweise ist ein zu Asn546 der SEQ ID NO: 1 korrespondierendes Asparagin N-glykosyliert, vorzugsweise mit einem Glykan der Strukturen nach Formel 1, 2, 3, 4, 5, 6, 7 oder 8.

Vorzugsweise ist ein zu Asn690 der SEQ ID NO: 1 korrespondierendes Asparagin N-glykosyliert, vorzugsweise mit einem Glykan der Strukturen nach Formel 1, 2, 3, 4, 5, 6, 7 oder 8.

In einem weiteren Aspekt betrifft die vorliegende Erfindung eine Präparation von rekombinanten ACE2 Polypeptiden, umfassend ein Polypeptid wie hierin definiert (ACE2 Monomere oder Dimere bzw. Monomer-Einheiten der Dimere), wobei der Anteil von ACE2 Polypeptiden mit einem scheinbaren Molekulargewicht, wie durch Gelelektrophorese bestimmbar, von unter 100 kDa oder von unter 101 kDa, bevorzugt von unter 104 kDa, speziell bevorzugt von unter 108 kDa, insbesondere von unter 112 kDa, besonders bevorzugt von unter 117 kDa, am meisten bevorzugt von unter 119 kDa, unter 20%, vorzugsweise unter 10%, insbesondere bevorzugt unter 5%, am meisten bevorzugt unter 1%, im Speziellen bei 0%, liegt, sowie alle Kombinationen hierfür. Z.B. können ACE2 Polypeptide von unter 100 kD zu 0% vorliegen von ACE2 Polypeptide unter 100 kDa unter 20% vertreten sein. Der Anteil bezieht sich auf alle der betroffenen ACE2 Formen und wird hierfür z.B. durch native Gelelektrophorese bestimmt.

Analog hierzu kann als Molmassenmaß die absolute Molmasse dienen welche durch Peptide-map festgestellt werden kann. So ist vorzugsweise der Anteil von ACE2 Polypeptiden mit einem Molekulargewicht von unter 86 kDa, oder von unter 89 kDa, bevorzugt von unter 92 kDa, speziell bevorzugt von unter 94 kDa, insbesondere von unter 97 kDa, besonders bevorzugt von unter 100 kDa, am meisten bevorzugt von unter 101 kDa, unter 20%, vorzugsweise unter 10%, insbesondere bevorzugt unter 5%, am meisten bevorzugt unter 1%, im Speziellen bei 0%, liegt, sowie alle Kombinationen hierfür. Z.B. können ACE2 Polypeptide von unter 86 kD zu 0% vorliegen; von ACE2 Polypeptide unter 97 kDa unter 20% vertreten sein.

Vorzugsweise liegt der Anteil von ACE2 Polypeptiden mit Transmembrandomänen unter 20%, vorzugsweise unter 10%, insbesondere bevorzugt unter 5%, am meisten bevorzugt unter 1%, im Speziellen bei 0%.

Vorzugsweise liegt der Anteil von ACE2 Multimeren unter 20%, vorzugsweise unter 10%, insbesondere bevorzugt unter 5%, am meisten bevorzugt unter 1%, im Speziellen bei 0%. Unter ACE2 Multimeren werden Komplexe mit 3 oder mehr ACE2 Polypeptiden verstanden. In einer Präparation von ACE2 Dimeren liegt zudem vorzugsweise der Anteil von ACE2 Monomeren unter 20%, vorzugsweise unter 10%, insbesondere bevorzugt unter 5%, am meisten bevorzugt unter 1%, im Speziellen bei 0%. In einer Präparation von ACE2 Monomeren liegt zudem vorzugsweise der Anteil von ACE2 Dimeren unter 20%, vorzugsweise unter 10%, insbesondere bevorzugt unter 5%, am meisten bevorzugt unter 1%, im Speziellen bei 0%.

Der Anteil an ACE2 Dimeren an ACE2 Molekülen beträgt mindestens 80% vorzugsweise mindestens 90%, 95% oder mindestens 99%.

Vorzugsweise ist der Anteil an ACE2 Polypeptiden mit N-glykosyliertem Asparagin entsprechend Asn53 der SEQ ID NO: 1 größer als 60%, vorzugsweise größer als 70%, insbesondere bevorzugt größer als 80%, speziell bevorzugt größer als 90%, am meisten bevorzugt größer als 99%, insbesondere 100%, und vorzugsweise mit einem Glykan der Strukturen nach Formel 1, 2, 3, 4, 5, 6, 7 oder 8.

Vorzugsweise ist der Anteil an ACE2 Polypeptiden mit N-glykosyliertem Asparagin entsprechend Asn90 der SEQ ID NO: 1 größer als 60%, vorzugsweise größer als 70%, insbesondere bevorzugt größer als 80%, speziell bevorzugt größer als 90%, am meisten bevorzugt größer als 99%, insbesondere 100%, und vorzugsweise mit einem Glykan der Strukturen nach Formel 1, 2, 3, 4, 5, 6, 7 oder 8.

Vorzugsweise ist der Anteil an ACE2 Polypeptiden mit N-glykosyliertem Asparagin entsprechend Asn103 der SEQ ID NO: 1 größer als 60%, vorzugsweise größer als 70%, insbesondere bevorzugt größer als 80%, speziell bevorzugt größer als 90%, am meisten bevorzugt größer als 99%, insbesondere 100%, und vorzugsweise mit einem Glykan der Strukturen nach Formel 1, 2, 3, 4, 5, 6, 7 oder 8.

Vorzugsweise ist der Anteil an ACE2 Polypeptiden mit N-glykosyliertem Asparagin entsprechend Asn322 der SEQ ID NO: 1 größer als 60%, vorzugsweise größer als 70%, insbesondere bevorzugt größer als 80%, speziell bevorzugt größer als 90%, am meisten bevorzugt größer als 99%, insbesondere 100%, und vorzugsweise mit einem Glykan der Strukturen nach Formel 1, 2, 3, 4, 5, 6, 7 oder 8.

Vorzugsweise ist der Anteil an ACE2 Polypeptiden mit N-glykosyliertem Asparagin entsprechend Asn432 der SEQ ID NO: 1 größer als 60%, vorzugsweise größer als 70%, insbesondere bevorzugt größer als 80%, speziell bevorzugt größer als 90%, am meisten bevorzugt größer alls 99%, insbesondere 100%, und vorzugsweise mit einem Glykan der Strukturen nach Formel 1, 2, 3, 4, 5, 6, 7 oder 8.

Vorzugsweise ist der Anteil an ACE2 Polypeptiden mit N-glykosyliertem Asparagin entsprechend Asn546 der SEQ ID NO: 1 größer als 60%, vorzugsweise größer als 70%, insbesondere bevorzugt größer als 80%, speziell bevorzugt größer als 90%, am meisten bevorzugt größer als 99%, insbesondere 100%, und vorzugsweise mit einem Glykan der Strukturen nach Formel 1, 2, 3, 4, 5, 6, 7 oder 8.

Vorzugsweise ist der Anteil an ACE2 Polypeptiden mit N-glykosyliertem Asparagin entsprechend Asn690 der SEQ ID NO: 1 größer als 60%, vorzugsweise größer als 70%, insbesondere bevorzugt größer als 80%, speziell bevorzugt größer als 90%, am meisten bevorzugt größer als 99%, insbesondere 100%, und vorzugsweise mit einem Glykan der Strukturen nach Formel 1, 2, 3, 4, 5, 6, 7 oder 8.

Vorzugsweise ist der Anteil an ACE2 Polypeptiden mit O-glykosyliertem Serin entsprechend Ser740 der SEQ ID NO: 1 größer als 60%, vorzugsweise größer als 70%, insbesondere bevorzugt größer als 80%, speziell bevorzugt größer als 90%, am meisten bevorzugt größer als 99%, insbesondere 100%.

Vorzugsweise ist die katalytische Aktivität des ACE2 Polypeptids oder der Präparation kkat mindestens 4 /s, vorzugsweise mindestens 5 /s, insbesondere bevorzugt mindesten 6 /s, speziell bevorzugt mindestens 7 /s, am meisten bevorzugt mindestens 7,6 /s, bezogen auf den Ang 1-7 (Angiotensin 1-7) Umsatz. Ang1-7 wird durch ACE2 aus AngII (Angiotensin II) gebildet. Der Umsatz kann wie in den Beispielen beschrieben auf simple Weise getestet werden. Dieser Umsatz bzw. die katalytische Aktivität von ACE2 kann auch aus anderen Assaydaten extrapoliert werden. Die Aktivität kann z.B. wie in der WO 2008/046125 A beschrieben gemessen werden.

Des Weiteren beschreibt die vorliegende Offenbarung ein Verfahren zur Herstellung von rekombinanten ACE2 Polypeptiden oder einer Präparation von rekombinanten ACE2, umfassend die Schritte der Einbringung eines ACE2 codierenden Polynukleotides, vorzugsweise eines ACE2 ohne Transmembrandomäne codierenden Polynukleotids, in eukaryotischen Zellen, Expression des ACE2 Polypeptids und Sammeln des exprimierten ACE2, insbesondere in dimerer Form. Die Zellen können danach selektiert werden, ein erfindungsgemäßes ACE2 Polypeptid, wie hierin beschrieben, insbesondere mit einem hohen Molekulargewicht, zu produzieren.

Vorzugsweise ist das ACE2 codierende Polynukleotid auf einem Vektor vorgesehen.

Vorzugsweise wird die ACE2-Expression mit einem Marker, vorzugsweise DHFR, selektiert. Vorzugsweise liegt der Marker auf dem Vektor vor.

Vorzugsweise weist der Vektor eine IRES für die exprimierte ACE2 mRNA auf (oder codiert hierfür).

Vorzugsweise sind die eukaryotischen Zellen CHO Zellen.

Die vorliegende Erfindung betrifft auch rekombinantes ACE2 Polypeptid oder eine Präparation von rekombinanten ACE2 Polypeptiden erhältlich durch ein solches Verfahren.

In einem weiteren Aspekt wird eine stabil mit einem ACE2 codierenden Polynukleotid transfizierte eukaryontische Zelllinie (oder Zelle), vorzugsweise CHO Zelllinie (oder Zelle), die ACE2, insbesondere wie oben definiert, exprimiert beschrieben. Die Zellline kann auf die gewünschten Eigenschaften, wie oben angegeben, z.B. Produktion von Dimeren aus Monomereinheiten mit einem Molekulargewicht, Von mind. 102 kDa, selektioniert werden. Die Zelle weist vorzugsweise eine ACE2-Produktivität von mindestens 10 pg/Zelle/Tag, vorzugsweise mindestens 15 pg/Zelle/ Tag, insbesondere bevorzugt von mindestens 20 pg/Zelle/Tag, auf.

Die Expression des ACE2 erfolgt vorzugsweise in Anwesenheit von genügend Zn²⁺-Ionen. Vorzugsweise kommen mindestens 0,5 micromolar, insbesondere bis zu 5 micromolar, Zn²⁺ zur Anwendung, insbesondere kann die Fermentation bei 2,5-3,5 micromolar Zn²⁺ durchgeführt werden. Die Zn²⁺-Konzentration z.B. im Nährmedium der exprimierten Zellen kann mindestens 0,5 µM, 0,75 µM, 1,0 µM, 1,25 µM, 1,5 µM, 1,75 µM, 2,0 µM, 2,25 µM oder mindestens 2,5 µM oder 3,0 µM sein. Weiteren Behandlungsschritte werden vorzugsweise ebenfalls in Anwesenheit von Zn²⁺-Ionen vorgenommen.

In einem weiteren Aspekt betrifft die vorliegende Erfindung Medikamente oder pharmazeutische Präparationen der erfindungsgemäßen ACE2 Produkts. Insbesondere zur Behandlung oder Vorbeugung von Bluthochdruck, Herzinsuffizienzen, wie kongestive, akute oder chronische Herinsuffizienz, Myokardinfarkt oder Artherosklerose, Nierenversagen oder -insuffizienz, Zystennieren (polycistic kidney disease, PKD) oder Lungenerkrankungen wie chronische obstruktive Lungenerkrankung, Pneumonie, Asthma, chronische Bronchitis, Lungenemphysem, zystische Fibrose, interstitielle Lungenerkrankung, pulmonale Hypertonie, Lungenembolie, Lungensarkoidose, Tuberculose, Lungenödem, ALI, ARDS oder Lungenkrebs. Allgemeine Behandlungsindikationen von ACE2 sind z.B. in der WO 2004/000367 A genannt, für die auch das erfindungsgemäße ACE2 Produkt geeignet ist.

Erfindungsgemäß kann eine pharmazeutische Zusammensetzung oder Medikament umfassend das ACE2 Protein zur Verfügung gestellt werden. Solche Zusammensetzungen können pharmazeutisch geeignete Salze derselben, zusätzlich Puffer, Tonizitätskomponenten oder pharmazeutisch geeignete Träger umfassen. Pharmazeutische Träger-Substanzen dienen der besseren Verträglichkeit der Zusammensetzung und ermöglichen bessere Löslichkeit sowie bessere Bioverfügbarkeit der Wirksubstanzen. Beispiele hierfür sind Emulgatoren, Verdickungsmittel, Redoxkomponenten, Stärke, Alkohollösungen, Polyethylenglycol oder Lipide. Die Auswahl eines geeigneten pharmazeutischen Trägers hängt stark von der Art der Verabreichung ab. Für orale Verabreichungen können flüssige oder feste Träger verwendet werden, für Injektionen sind flüssige Endzusammensetzung erforderlich.

Vozugsweise umfasst die erfindungsgemäße ACE2 Zusammensetzung Puffersubstanzen oder tonische substanzen. Mittels Puffer kann der pH-Wert des Medikaments auf physiologische Konditionen eingestellt werden und weiters können pH-Schwankungen abgeschwächt bzw. gepuffert werden. Ein Beispiel hierfür ist ein Phosphatpuffer. Tonische Substanzen dienen zur Einstellung der Osmolarität und können ionische Substanzen, wie zum Beispiel anorganische Salze, wie NaCl oder KCl, oder auch nicht-ionische Substanzen, wie zum Beispiel Glycerin oder Kohlenhydrate, umfassen.

Bevorzugterweise ist die erfindungsgemäß zu verwendende Zusammensetzung oder das Medikament zur systemischen, topischen, oralen oder intranasalen Verabreichung oder als Inhalationsverabreichung geeignet hergerichtet. Diese Verabreichungsformen der Zusammensetzung der vorliegenden Erfindung ermöglichen eine schnelle und unkomplizierte Aufnahme. Sofern die ACE2 Zusammensetzung zur oralen Verabreichung bestimmt ist, wird es vorzugsweise in einer Magensaftresistenten Formulierung oder Verkapselung vorgesehen. Zur oralen Verabreichung können beispielsweise feste bzw. flüssige Medikamente direkt oder aufgelöst bzw. verdünnt eingenommen werden.

Das erfindungsgemäß zu verwendende Medikament ist vorzugsweise zur intravenösen, intraarteriellen, intramuskulären, intravaskulären, intraperitonealen oder subkutanen Verabreichung geeignet hergerichtet. Hierfür eignen sich beispielsweise Injektionen oder Transfusionen. Verabreichungen direkt in die Blutbahn haben den Vorteil, dass die Wirkstoffe des Medikaments im gesamten Körper verteilt werden und die Zielgewebe schnell erreichen.

Die vorliegende Erfindung wird durch die folgenden Figuren und Beispiele weiter illustriert ohne auf diese beschränkt zu sein.

### Figuren:

Fig.1: ACE2 Expressions- und Selektionskassette
Fig.2: ACE2 spezifische Western Blot Analyse der Produktionsklongeschichte
Fig.3: SDS-PAGE Analytik des ACE2 Monomers
Fig.4: Klonauswahl

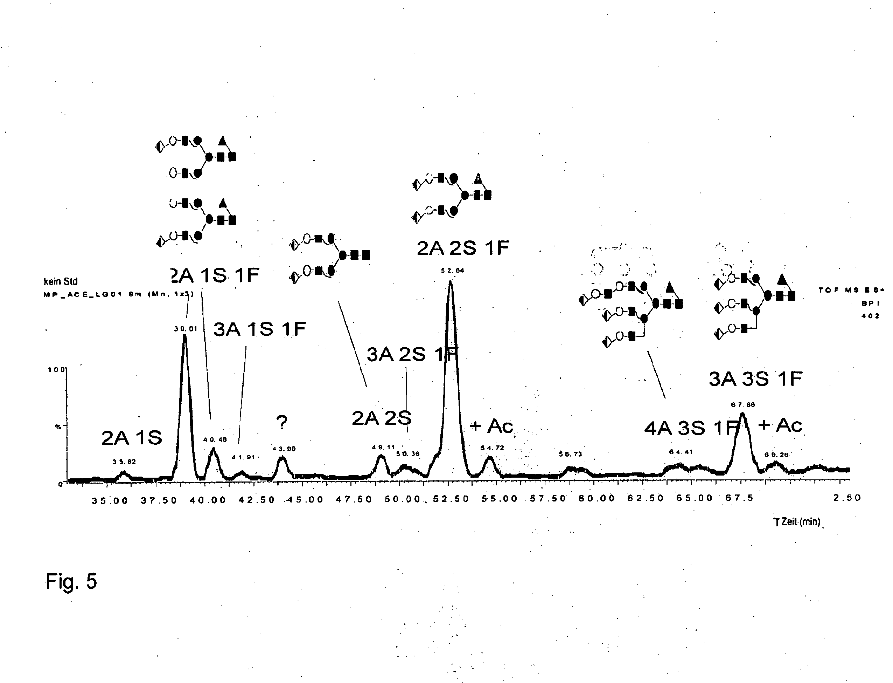

Fig.5: LC-MS Glykosylierungsanalytik
Fig.6: Präparative Größentrennung von ACE2
Fig.7: Pharmakokinetik von ACE2 in 3 Spezies
Fig.8: Kalibrationskurven zur Ang 1-7 und Ang II Quantifizierung.
Die Peptide wurden im angeführten Konzentrationsbereich mittels RP-HPLC auf Waters C18 µbondapak RP, 2,1x300mm, 10µm, 125Ǻ Säule aufgetrennt.

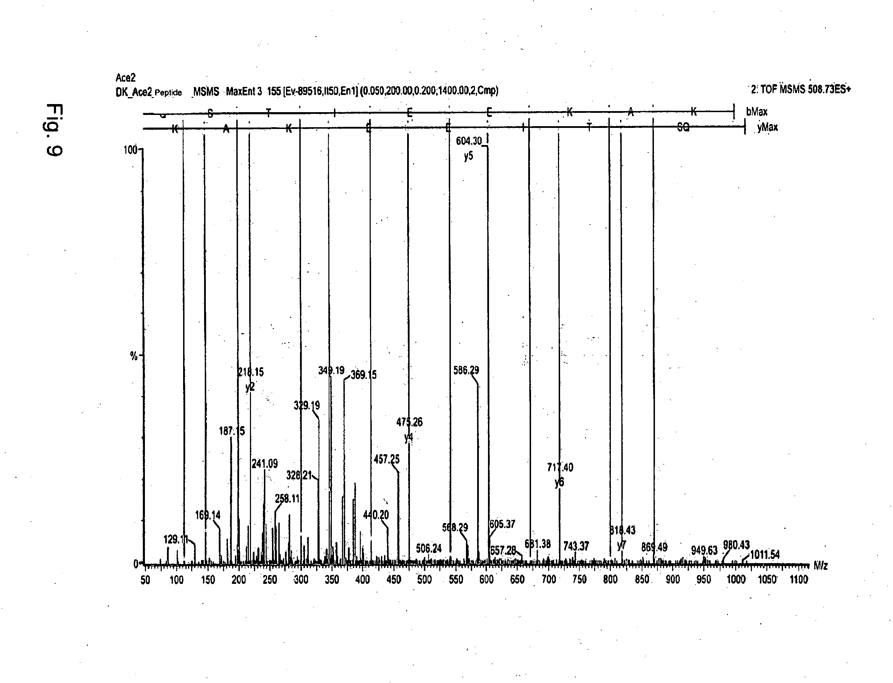

Fig. 9: MS/MS-Spektrum des N-terminalen Peptids. Anmerkung: Q und K haben die gleiche Masse.
Fig. 10: Sequenz von ACE2 und N-Glykosylierungs-Vorhersage
Fig. 11: aufgeschlüsseltes Spektrum für Glykosylierungsstellen 103 (A), Stelle 432 (B), Stelle 546 (C), Stelle 690 (D), Stelle 90 (E).

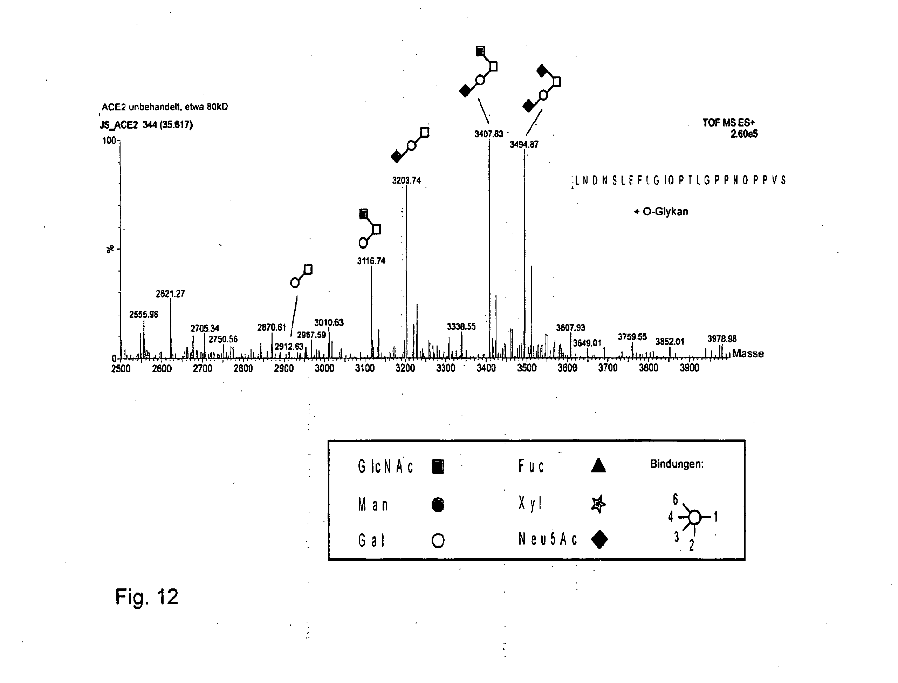

Fig. 12: Spektrum des C-terminalen O-glykosylierten Peptids. Die strukturelle Zuordnung muss als probeweise klassifiziert werden.
Fig. 13: LC-MS freigesetzter Glykane.
Fig. 14: Bestimmung der ACE2 Dimerstruktur mittels nativem PAGE (links, Proteinbanden wurden mittels Silberfärbung visualisiert) und SEC (rechts, Trennung auf einer Zorbax G-450 Säule in Gegenwart von 220 mM Na-Phosphat bei pH 7.0 in 10% Acetonitril, das Chromatogram wurde bei 214 nm aufgenommen.
Fig. 15: Chromatogramm einer Größenausschlusschromatographie des Dimeren ACE2 (Retention 8,55 min, 8,93 min). Standard: Thyroglobulin (670 kDa, 7,43 min), Gamma-globulin (158 kDa), Ovalbumin (43 kDa, 10,08 min), Myoglobulin (17 kDa, 11,08 min), Vitamin B-12 (1,3 kDa, 12,71 min).
Fig. 16: ACE2 spezifische Western Blot Analytik von Zellextrakten aus Cortex (A), Gehirn (B) und einem ACE2-Dimer Expressionsklon (C). In D wurde ein reines ACE2-Dimer aufgetragen.
Fig. 17: Analytisches SEC-HPLC Chromatogram der ACE2 Monomerform. Laufbedingungen: Säule: Zorbax GF250, Puffer: 220 mM Na₂H-PO₄ + 10% CH₃CN, pH 8.0 bei 1 ml/min.
Fig. 18: PAGE Analytik von ACE2-Dimer (A) und ACE2 Monomeren (B), Proteine wurden mittels Silberfärbung (a) und ACE2 spezifischem Western-blot (b) nachgewiesen.
Fig. 19: Bestimmung der enzymatischen Aktivität von ACE2 Monomeren im Vergleich zu ACE2-Dimer. Es wurden bei konstanter Anfangskonzentration des fluorenzenzmarkierten Substrates Cumarin-APK-DNP und vier unterschiedliche Enzymkonzentrationen eingesetzt und die jeweiligen Fluoreszenzkurven verglichen.
Fig. 20: ACE2 Serumaktivität gemessen 24 und 48 Stunden nach ACE2-Dimer-Gabe (2,5 mg/kg, blaue Balken) oder ACE2 Monomerform-Gabe (2,5 mg/kg, graue Balken)

### Beispiele:

### Beispiel 1: Expression von hochglykosyliertem ACE-Dimer

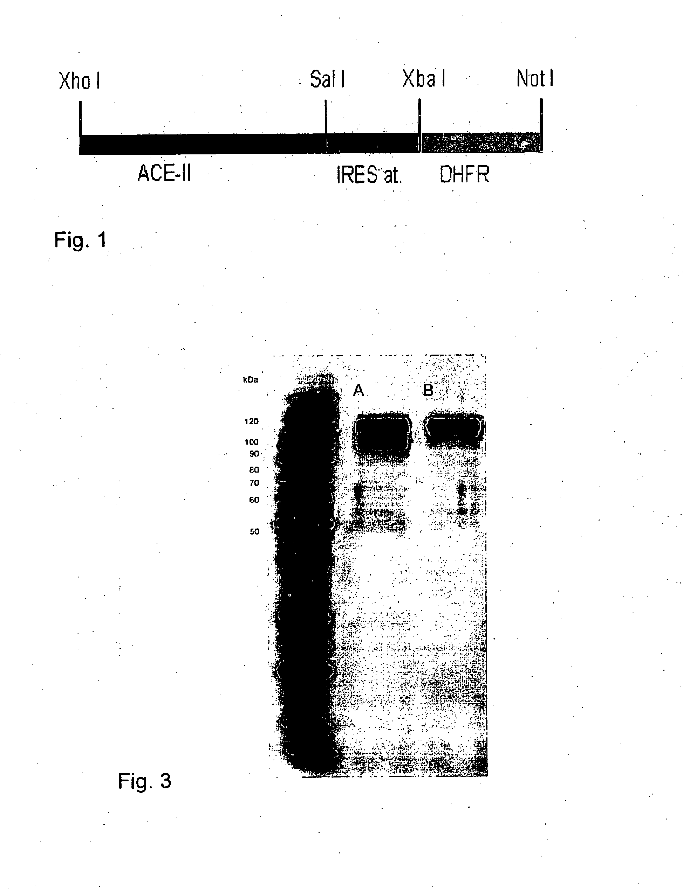

Der lösliche Abschnitt der humanen ACE2 Sequenz (SEQ ID NO:1) wurde in einen Expressionsvektor kloniert, in welchen zuvor der amplifizierbare Selektionsmarker DHFR eingefügt wurde, um zu einer erhöhten Expression des ACE2 Genes zu führen. Hierzu wurde zwischen die für ACE2 und DFHR kodierenden Gene eine attenuierte IRES eingebracht, welche die bi-cistronische Ablesung von ACE2 und DHFR auf derselben mRNA ermöglicht. Die ACE2 Expressions- und Selektionskassette ist in Figur 1 graphisch dargestellt. Nachdem beide Proteine unter Kontrolle desselben Promotors exprimiert werden, kann über DHFR Selektion unter Verwendung des Antagonisten MTX zielstrebig die ACE2 Expression gesteigert werden. Durch diese Strategie ist es möglich, besonders stabile Expressionszelllinien zu erhalten, die hohe Ausbeuten eines Produktes konstanter Qualität liefern. Dies ermöglicht es auch in Zelllinien vernünftige Produkttiter zu erzielten, die möglicherweise für die rekombinante Expression eines bestimmten Targetproteins minder geeignet sind.

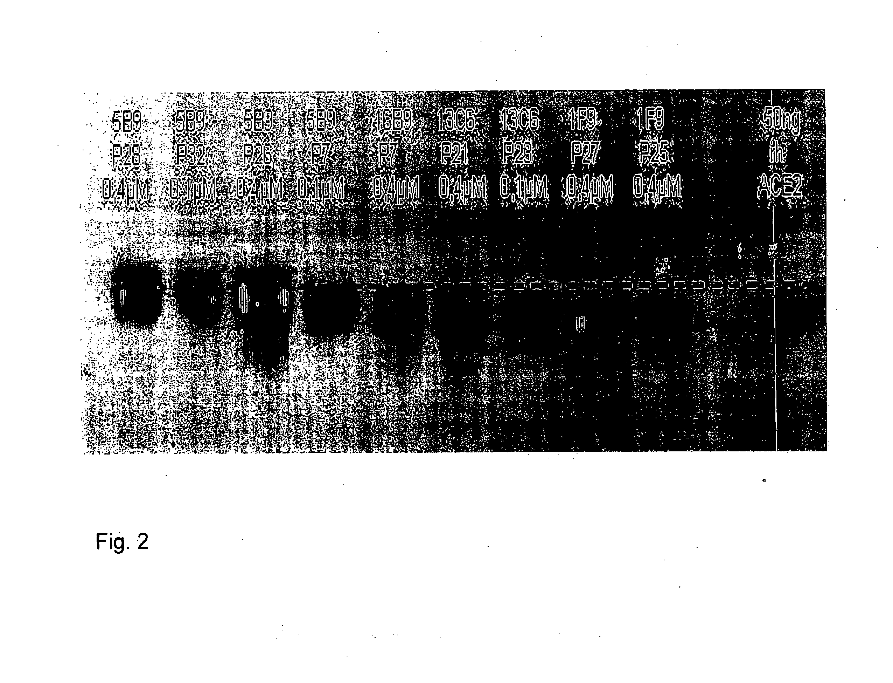

Dieser Vektor wurde in CHOdhfr- Zellen transfiziert und unter kontinuierlich gesteigertem MTX Druck wurde die Kopienanzahl der ACE2 Gene amplifiziert. Über mehrere Selektions- und Subklonierungsrunden wurden die besten Produzenten mittels intrazellulärer FACS Analytik und proteinchemischer sowie enzymatischer Analysen auf optimale Produkteigenschaften selektiert: Es wurden für die Auswahl des bestgeeigneten Klons vor allem die spezifische enzymatische Aktivität, welche mit 3 verschiedenen Substraten gemessen wurde, die Produkthomogenität, die zelluläre Produktivität, jedoch auch die Zuckerkomplexizität in Betracht gezogen. In Fig. 2 ist eine rekapitulative Western Blot Analyse der sukzessiven Kulturüberstände einzelner Klone dargestellt, die zur Etablierung der Produktionszelllinie verwendet wurden. Die Produkteigenschaften der einzelnen Klone unterscheiden sich insofern, dass der Zuckeranteil der Expressionsprodukte von rechts nach links zunimmt, was durch eine deutliche Massenzunahme erkennbar ist. Dies wurde durch spezifische Selektion hochglykosylierender Klone erreicht. Letztendlich wurde ein Klon (Klon 5B9), welcher das Expressionsprodukt mit dem höchstem Molekulargewicht aufwies, zur Etablierung der Produktionszelllinie (1B4) verwendet.

Es wurde entschieden, 6 Klone weiterzuführen, die enzymtisch hoch aktives und komplex N-glykosyliertes ACE2 exprimierten. Während lösliches ACE2 ein Molekulargewicht von 83 kDa aufweist, werden Klone ausgewählt, welche im denaturierenden SDS-PAGE, bedingt durch deren Zuckerstruktur, im Bereich von bis zu 120 kDa erschienen. Fig. 3 zeigt ein durch den vorliegenden Produktionsprozess hergestelltes ACE2 (Lane B) im Vergleich zu einem in NS0 hergestellten ACE2 Standard (Lane A). Während das erfindüngsgemäße ACE2 Polypeptid - hier als Monomer analysiert - homogen, hoch glykosyliert ist und daher als einzelne Bande bei ca. 120 kDa erscheint weist das Referenzmaterial Banden von 83 kDa bis zu 120 kDa auf, was eine sehr heterogene Glykosylierung andeutet.

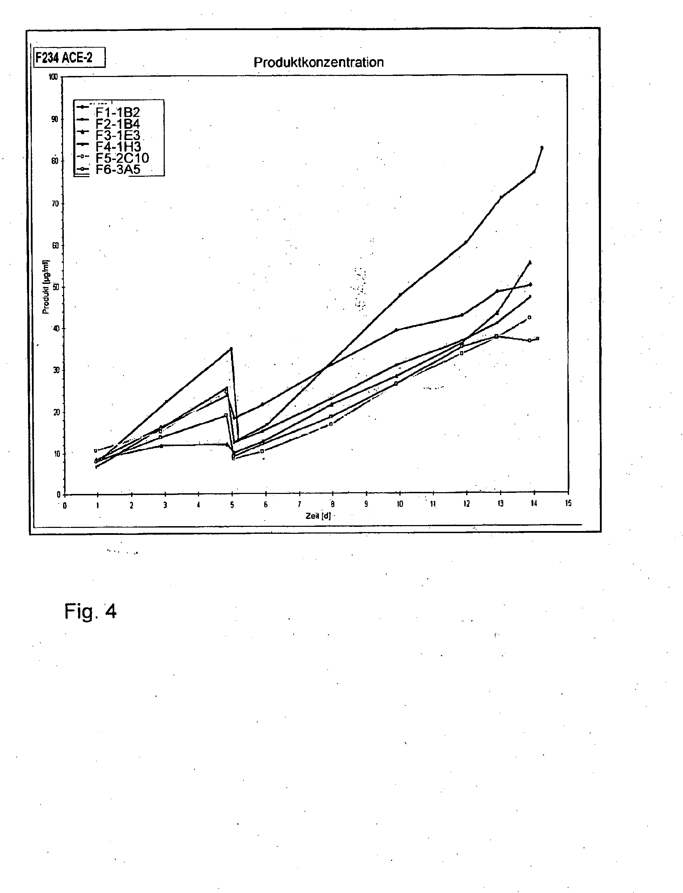

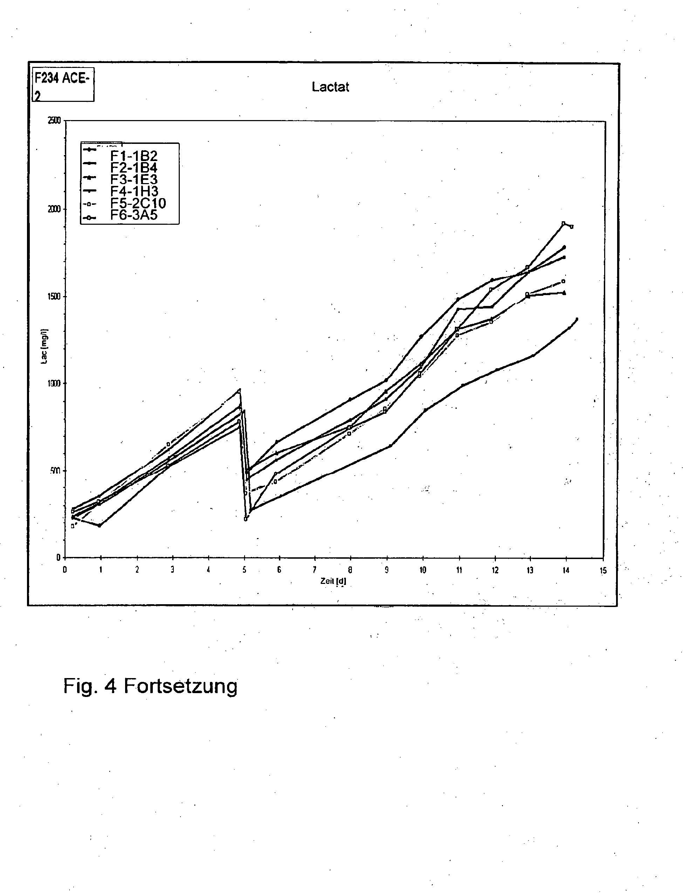

Die präliminären Klone wurden im Weiteren auf proteinfreies Wachstumsmedium (Polymun) umgestellt. Dieses käufliche Medium ist chemisch definiert, Serum-frei, frei von tierischen Proteinem und auf die rekombinante Expression von Glykoproteinen in CHO optimiert. Die Fermentation wurde bei 2,5-3,5 µM Zn²⁺ durchgeführt. Alle 6 Klone wurden in Kultur gehalten und auf deren Produktionsprozesstauglichkeit überprüft. Im Speziellen wurden Wachstumsraten aufgezeichnet sowie die Überstände auf Produktverlauf und Metaboliten untersucht (Fig. 4). Wiederum wurden die Expressionsprodukte sowie die Klone genau analysiert. Alle Klone exprimierten hochaktives ACE2 und wiesen Produktivitäten um 20-30 pg/Zelle/Tag auf. Weiters wurden ebenfalls die Zuckerstrukturen und deren Heterogenität analysiert. Letztendlich wurde Klon 1B4 ausgewählt. Er wies über den gesamten Produktionsverlauf eine homogene Zuckerstruktur auf. Es waren alle 7 N-Glykosylierungsstellen prozessiert, wobei diese mindestens eine bi-, manche jedoch auch eine tri-antennäre komplexe Glykosylierung mit terminalen Sialinsäuren aufwiesen.

Auf der Basis dieses Klones wurde eine Mastercellbank hergestellt und getestet, sowie ein GMP-tauglicher Reinigungs- und in weiterer Folge ein GMP Produktionsprozess aufgebaut.

SEQ ID NO: 1 (ACE2 Proteinsequenz, die autologe Signalsequenz (unterstrichen) wird durch die Wirtszelle für die Ausschleusung abgespaltet).
MSSSSWLLLSLVAVTAA

Bedingt durch die Dimerisierung von ACE2 sind sämtliche hydrophobe Proteineinheiten in das Innere des Komplexes gerichtet, wobei die geladenen Reste, wie N-gebundenen Zuckerketten nach außen ragen und die Struktur im ebenfalls geladenen physiologischen Milieu solvatisieren. Diese Dimerisierung durch Expression eines vollständig N-glykosylierten ACE2 wurde in Gegenwart von Zn²⁺ festgestellt. Der Dimer-Komplex besteht hierbei aus 2 identischen Untereinheiten, die elektrostatisch aneinander gebunden sind und sich auch nicht mehr in physiologischen Lösungen trennen. Es kommt hierbei zur Sekretion eines Glykoproteins mit jeweils 14 stark geladenen Sialinsäurestrukturen auf jedem ACE2 Molekül sowie 28 Sialinsäurestrukturen im Dimer. Jeweils 2 Zn²⁺ Ionen werden in den Komplex eingebaut und stabilisieren dessen Struktur. Die starke Ladung der Zuckerketten solvatisiert das Molekül in physiologischen wäßrigen Lösungen und zwingt die zugehörigen geladenen Proteindomänen nach außen. Der Produktionsprozeß wurde so aufgebaut, dass im Endprodukt ausschließlich ACE2 Dimere vorkommen.

Dies wird dadurch ermöglicht, dass bei Generierung des ACE2 genügend Zn²⁺-Ionen vorhanden sind (vorzugsweise kommen 1,5-5 micromolar Zn²⁺ zur Anwendung, insbesondere kann die Fermentation bei 2,5-3,5 uM Zn²⁺ durchgeführt werden) und anschließend die weiteren Behandlungsschritte in Anwesenheit von Zn²⁺-Ionen vorgenommen werden.

Die Aufreinigung erfolgte unter Verwendung eines Anionentauschschrittes, einer Ammoniumsulfatpräzipitation, eines HIC-Schrittes, eines Kationentauscherschrittes sowie eines weiteren hochauflösenden Anionentauscherschrittes. Alle Medien im Prozeß waren phosphatgepuffert und enthielten Natriumchlorid. Der finale Probenpuffer ist eine physiologische Glycinlösung zur Injektion.

### Beispiel 2: Produkteigenschaften

Komplexes, hochglykosylieres ACE2 Monomer bzw. Monomereinheiten des Dimers weisen aufgrund der kovalent gebundenen Zuckerstrukturen ein deutlich höheres Molekulargewicht auf als es der Aminosäuresequenz entspricht. So wurde mittels Peptidemap eine Molmasse von 102 kDa gemessen, während die von nichtglykosyliertem ACE2 nur 83 kDa beträgt. Der Zuckeranteil liegt dementsprechend bei 23% und ist für die im Weiteren beschriebenen Produkteigenschaften von essentieller Bedeutung. Aufgrundstarker Hydratisierung erscheint die Monomereinheit in der SDS-PAGE bei ca. 120 kDa (Fig. 3) im Vergleich zu den Referenzstandards.

Nicht das natürliche, membrangebundene ACE2 wurde exprimiert, sondern ausschließlich der lösliche Anteil von ACE2. Aufgrund der fehlenden Membrandomäne wird es vollständiger glykosyliert.

Aufgrund der komplexen, hoch-sialylierten und somit stark negativ geladenen Zuckerstrukturen besitzt ACE2 eine deutlich höhere Löslichkeit im Vergleich zu nicht glykosyliertem oder unvollständig glykosyliertem ACE2. Somit können physiologisch gepufferte Proteinformulierungen von bis zu 15 mg/ml problemlos hergestellt werden.

Das Produkt verhält sich ferner auch stabil in Lösung und verliert über einen längeren Lagerungszeitraum weder Aktivität, noch neigt es zum Abbau oder zur Aggregation, abgesehen von der stabilen Dimerisierung. Deglykosyliertes ACE2 hingegen fällt bereits im geringen Konzentrationsbreich aus. Dies konnte in einem präparativen Deglykosylierungsansatz mittels PNGaseF gezeigt werden. Der für ACE2 berechnete, theoretische Stabilitätsindex beträgt 41,2 und klassifiziert das Protein als instabil, wobei bei dieser Berechnung die Zuckerstrukturen außer Acht gelassen wurden. Nachdem sich die Formulierungen jedoch monatelang in Lösung stabil verhalten, ist dies offensichtlich auf den hohen Zuckeranteil zurückzuführen. Die bessere Solvatisierung von ACE2 führt weiters dazu, dass diese Formulierung ausschließlich aus ACE2 Dimeren (bzw. nach künstlicher Trennung zum Monomer ausschließlich aus Monomeren) besteht, während nicht glykosyliertes ACE2 zur Multimerisierung und zur Aggregation neigt.

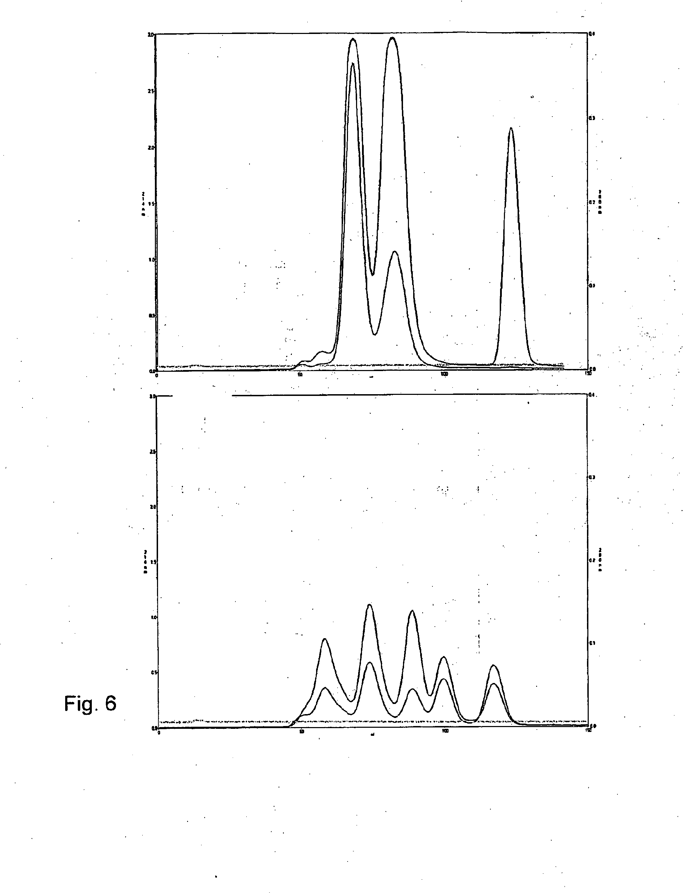

Aufgrund des hohen Anteils geladener Zuckerreste nimmt weiters der hydrodynamische Durchmesser, die Solvatisierungshülle der vorliegenden ACE2 Präparation stark zu. Dieser Umstand kann zur präparativen Aufreinigung von ACE2 über Größentrennungssäulen genutzt werden, wo das hier ausschließlich als Dimer vorliegende Protein bei einem scheinbaren Molekulargewicht von 250 kDa im Vergleich zu den berechneten 83 kDa erscheint. Ein Chromatogramm einer präparativen ACE2 Reinigung ist in Fig. 6 wiedergegeben. ACE2 (erste Abbildung) eluiert mit einer Retentionszeit von 69 Minuten. Dies entspricht einem scheinbaren Molekulargewicht zwischen dem von Thyroglobulin (erste Abbildung bei 58 Minuten, 670 kDa) und Gamma-Globulin (erste Abbildung bei 74 Minuten, 158 kDa). In diesem Bereich hoher Molekulargewichte kommen in Kulturüberständen so gut wie keine Kontaminationen vor, sodass hochreines Produkt in einem sehr einfachen Trennungsschritt abgetrennt werden kann.

### Beispiel 3: Pharmakologische Produkteigenschaften

Die erfindungsgemäße ACE2 Präparation liegt als stabile, hochreine und konzentrierte Proteinlösung in physiologischer Pufferung vor und kann ohne weitere Stabilisierung gelagert und verabreicht werden. Z.B. kommt ein Phosphatpuffer zum Einsatz.

ACE2 ist als Monomer oder Dimer stabil in Lösung und zeigt aufgrund des hohen Zuckeranteils keine Aggregation. Ferner weist die ACE2 Präparation die volle enzymatische Aktivität auf.

ACE2 kann aufgrund seiner Löslichkeit als Bolus i.v. verabreicht werden. Die Bioverfügbarkeit ist aus denselben Gründen systemisch sofort nach Gabe gewährleistet.

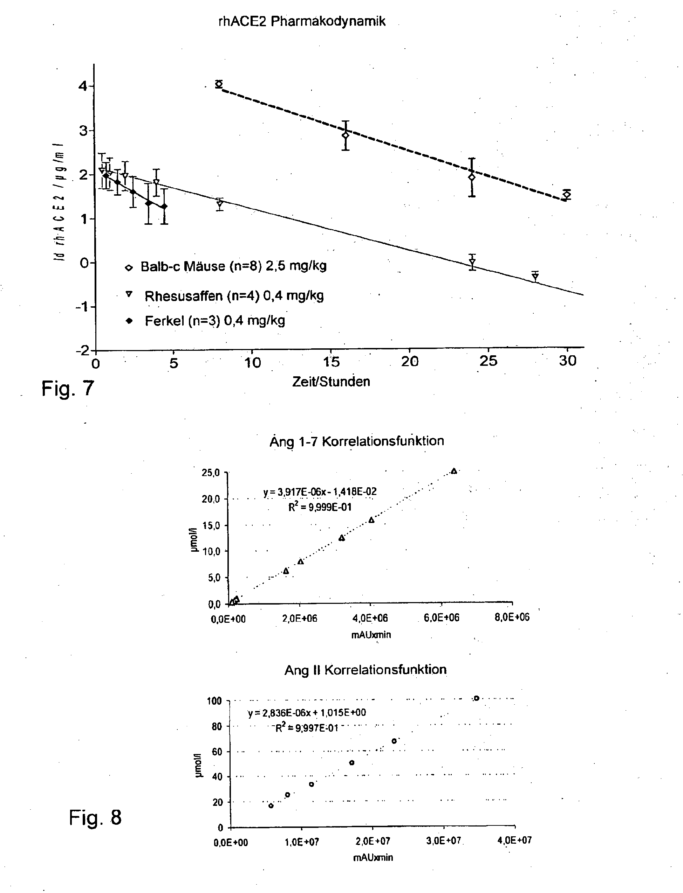

Aufgrund des hohen, stark verzweigten und komplexen Zuckeranteils wird ACE2 nur langsam abgebaut. Es resultiert daraus eine lange Halbwertszeit von mindestens 10.5 Stunden, welche in diversen Spezies, vor allem auch in Rhesus Makaken gemessen wurde. Fig. 7 stellt die gemessene Halbwertszeit von i.v. gegebenem ACE2 in 3 Spezies dar.

Der hohe Sialinsäureanteil bedingt weiters, dass gegen ACE2 keine neutralisierende Immunantwort aufgebaut wird. Diese wäre nicht nur für die exogene Gabe von ACE2 kontraproduktiv, sondern könnte auch autologes, zellständiges ACE2 neutralisieren.

Die beschriebene ACE2 Formulierung mitsamt sämtliieh-et einhergehender Produkteigenschaften ermöglicht daher erst eine effiziente Therapie mit rhACE2.

### Beispiel 4: Bestimmung der spezifischen ACE2 Aktivität

Die spezifische Aktivität von ACE2 Präparationen wurde durch Messung des Umsatzes von Ang II (Asp-Arg-Val-Tyr-Ile-His-Pro-Phe) bestimmt. Sämtliche Messungen wurden als Dreifachbestimmungen in einem Ansatzvolumen von 100 µl durchgeführt. Die enzymatische Reaktion wurde durch Zugabe von 250 ng/ml ACE2 zu einer 80 µM Ang II Lösung in 50 mM MES, 300 mM NaCl, 10µM ZhCl₂ und 0,01% Brij-30 bei pH 6,5 gestartet. Die Proben wurden sorgfältig gemischt und für genau 18 Minuten bei 37°C inkubiert. Die enzymatische Reaktion wurde durch Zugabe von 100 mM EDTA gestoppt. Zur Analyse wurden die Lösungen mittels RP-HPLC (Waters C18 µBondapak, 2,1x300mm, 10µm, 125Ǻ) unter Verwendung eines linearen Gradienten von 10 bis 60% CH₃CN in 0,08% H₃PO₄ über 20 Minuten bei einer Flussrate von 1 ml/min aufgetrennt. Weiters wurden sowohl Ang II als auch Ang 1-7 Peaks in den Chromatogrammen erkannt und integriert. Die Peptidkonzentrationen wurden anhand der jeweiligen in Fig. 8 dargestellten Kalibrationskurven ermittelt. Weit ters wurde der enzymatische Umsatz sowie die spezifische Enzymaktivität bestimmt.

Die Aktivität des ACE2 Produktes wurde wie zuvor beschrieben bestimmt. In Tabelle 1 sind die jeweiligen Resultate der Peakintegration sowie die berechneten Enzymdaten dargestellt.

Tabelle 1: Enzymdaten und Reaktionsbedingungen. Es sind die Mittelwerte von Dreifachbestimmungen angegeben.

| ***Enzymatische Reaktivität*** | | | | | |
|---|---|---|---|---|---|
| ***Peak Fläche*** | ***Umsatz*** | ***Reaktionszeit*** | ***A CE2 Konz.*** | ***kkat*** | ***Spezif. Aktivität*** |
| mAU.min | µmol.ml⁻¹ | min | ng.ml⁻¹ | s⁻¹ | µmol.mg⁻¹.min⁻¹ |
| **Ang II** | | | | | |
| 17149890 | 62,1 | 18 | 250 | 8,0±0,3 | 4,7±0,2 |
| **Ang 1-7** | | | | | |
| 4720141 | 23,1 | 18 | 250 | 8,8±0,2 | 5,2±0,1 |

Die ACE2 Präparation weist eine katalytische Aktivität kkat von 8,0+0,3 /s gemessen anhand des Ang II Umsatzes und 8,8±0,2 / s in Bezug auf den Ang 1-7 Umsatz auf. Beide Werte stimmen gut überein und sind deutlich höher als die Angaben von Vickers et al., (J Biol Chem. 277 (17) (2002):14838-43), wo eine katalytische ACE2 Aktivität von 3,5 /s veröffentlicht wurde. Die Reaktionbedingungen waren identisch. Die Ursache der 240% höheren Aktivität unserer Präparation dürften post-translationale Modifikationen und hier vor allem die N-Glykosylierung sein, die in dem Material, das Vickers verwendet hat, deutlich geringer ausgeprägt war. Dieses Material wurde in Insektenzellen exprimiert und wies zwar dieselbe Aminosäuresequenz auf, war jedoch zu einem deutlich geringeren Anteil und Verzweigungsgrad glykosyliert. Es wurde weiters auch eine im Handel erhältliche ACE2 Präparation von R&D systems (cat. 933-eN), die ebenfalls eine deutlich geringere Aktivität kkat von 2,0±0,1 /s aufwies, untersucht. Eine wesentliche Eigenschaft der erfindungsgemäßen Präparation ist die besonders hohe enzymatische Aktivität, die vor allem durch post-translationale Modifikationen ermöglicht wird.

### Beispiel 5: Glycoproteomische Analyse von rekombinantem ACE2

Die Probe von gereinigtem, CHO-exprimiertem ACE2 wurde auf zweierlei Arten analysiert:
Erstens wurden tryptische Peptide nach SDS-PAGE und S-Alkylierung erzeugt und mittels LC-ESI-MS (und MS-MS) analysiert. Das N-terminale Peptid und mehrere interne Peptide wurden gefunden. Fünf der sieben potentiell N-glykosylierten Stellen wurden in glykosylierter Form mit Glykanstrukturen gefunden, die hauptsächlich di-antennäre Glykane mit Fucose und verschiedene Mengen an Sialinsäure enthielten. Das C-terminale Peptid scheint O-glykosyliert zu sein.

Zweitens wurden freie, reduzierte N-Glykane mittels Carbon-LC-ESI-MS analysiert. Für das di-antennäre, di-sialysierte Glykan mit Fucose konnte gezeigt werden, dass die Fucose mit dem Kern α1,6-verbunden ist und die Sialinsäure α2,3-verbunden ist. Zusätzlich zu mono- oder di-sialysierten, di-antennären Glykanen wurde eine beträchtliche Menge an tri-antennärem Oligosaccharid gefunden. Eine grobe Schätzung der Masse des glykosylierten ACE2 ergibt 101-102 kDa, d.h. bestehend aus etwa 17% Kohlehydrat.

### Proteolytischer Verdau von mittels SDS-PAGE getrennten hBChE

Aliquots von ACE2 wurden einer SDS-PAGE unterzogen, entfärbt, carbamidomethyliert, mit Trypsin von Sequenzier-Qualität verdaut und aus Gel-Stücken, wie beschrieben, extrahiert. Die Extrakte wurden in einem Speed Vac-Konzentrator zur Trockene gebracht und mit Wasser, das 0,1% Ameisensäure enthielt, vor der nachfolgenden LC-MS-Analyse rekonstituiert. Für die Oligosaccharid-Analyse wurden die Peptide mit PNGase F verdaut, und die Glykane wurden unter Verwendung von C18 SPE-Spitzen gereinigt.

### MS-Analyse tryptischer Peptide und Glykopeptide

Die massenspektrometrische Analyse wurde auf Q-TOF Ultima Global (Waters Micromass), ausgerüstet mit einer Standard-Elektrosprüheinheit, einem Cap-LC-System (Waters Micromass) und einem 10-Port-Lösungsmittel-Wechsel-Modul (Rheodyne), wie kürzlich beschrieben [Kolarich 2006], durchgeführt. Die Proben wurden zuerst auf einer Aquasil C18-Vorsäule (30 x 0,32 mm, Thermo Electron) unter Verwendung von Wasser als Lösungsmittel eingefangen. Die Analyse-Säule, eine Biobasic C18-Säule (100 x 0,18 mm, Thermo Electron) wurde vor dem Lösungsmittel-Wechsel auf 5% Acetonitril gehalten und danach wurde ein linearer Gradient von 5 bis 50% Acetonitril mit einer Flussrate von 2 µl/min angelegt. Alle Elutionsmittel enthielten 0,1% Ameisensäure.

Die Proben wurden auf MS- und auch auf MS-MS-Art analysiert. Die Datenanalyse erfolgte mit MassLynx 4.0 SP4 software (Waters Micromass).

### Analyse freier N-Glykane von ACE2

Borhydrid-reduzierte Glykane wurden auf einer porösen graphitischen Kohlenstoff-Säule getrennt und mittels Massenspektrometrie nachgewiesen.

### Beispiel 6: Glykosylierungsanalytik

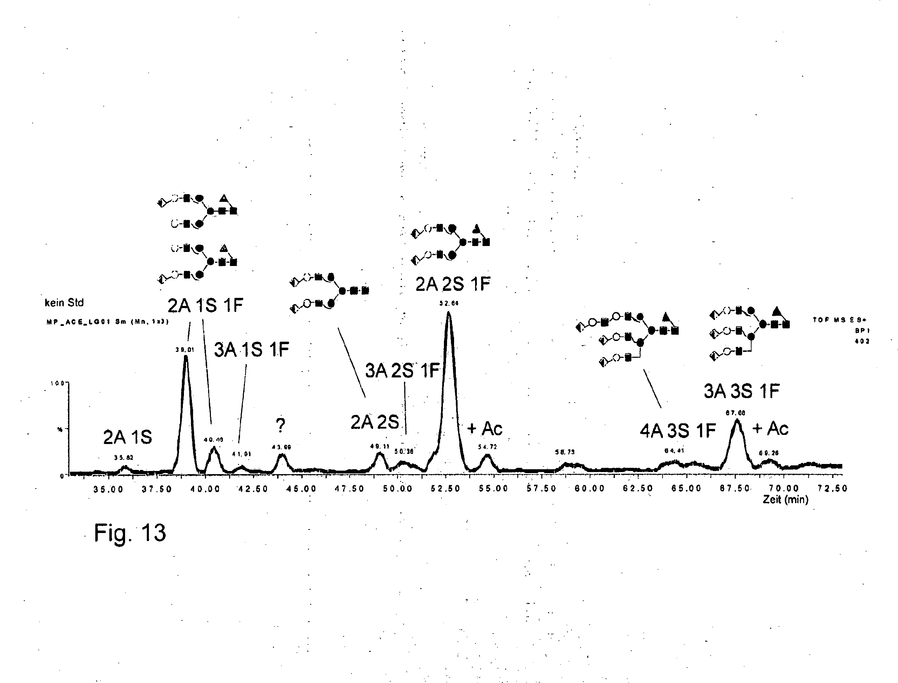

Die Proteinsequenz von ACE2, dessen Molekulargewicht, die Position sämtlicher Glykosylierungsstellen sowie die Struktur der gebundenen Zucker wurden hierin bestimmt. Die Probe wurde mittels tryptischem Verdau, S-Alkylierung und LC-ESI-MS analysiert.
- Die ermittelte Proteinmasse des glykosylierten Produktes beträgt 102 kDa, wobei der Zuckeranteil 23% der Gesamtmasse entspricht.
- Der N-terminus sowie die Anwesenheit sämtlicher sequenzinternen Peptide wurden hierbei bestätigt (siehe Sequenzinformation).
- Alle 7 postulierten N-Glykosylierungsstellen (Positionen 53, 90, 103, 322, 432, 546 und 690) enthielten tatsächlich di-antennäre, komplexe sialinsäurehältige fukosylierte Zuckerstrukturen (Fig. 13)
- Das C-terminale Peptid war O-glykosyliert.

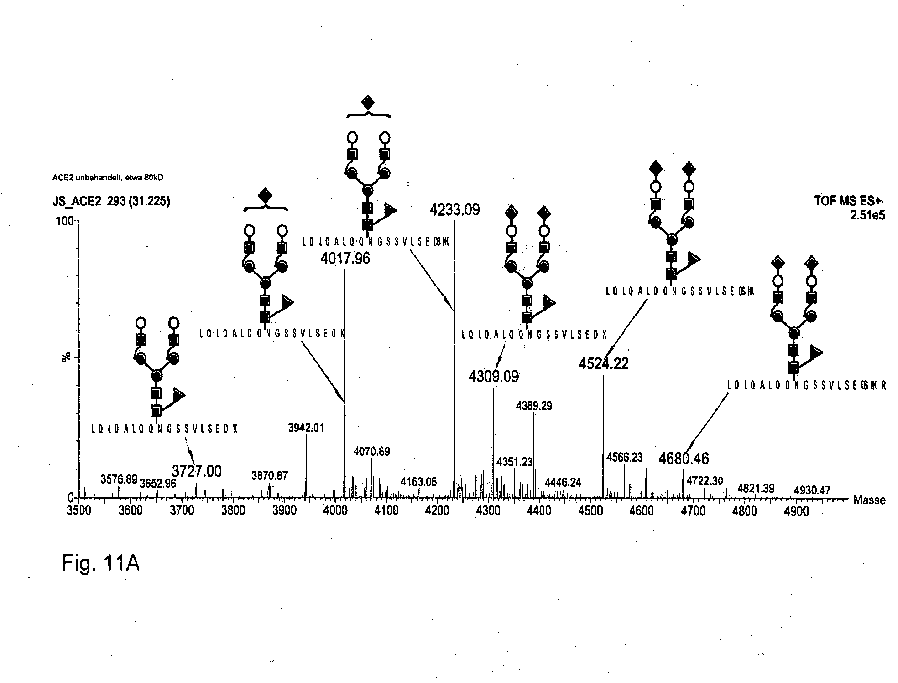

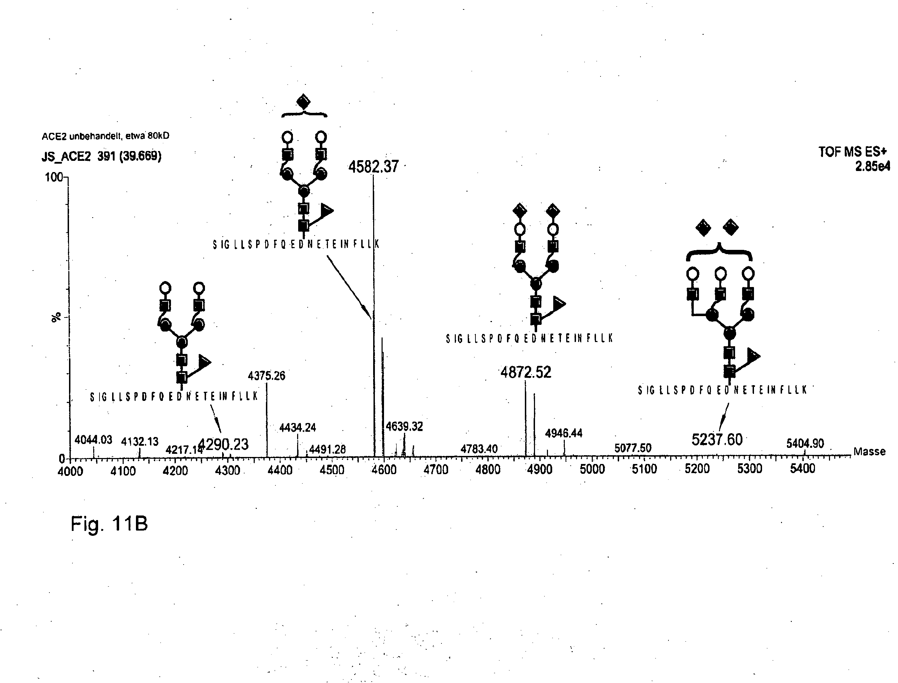

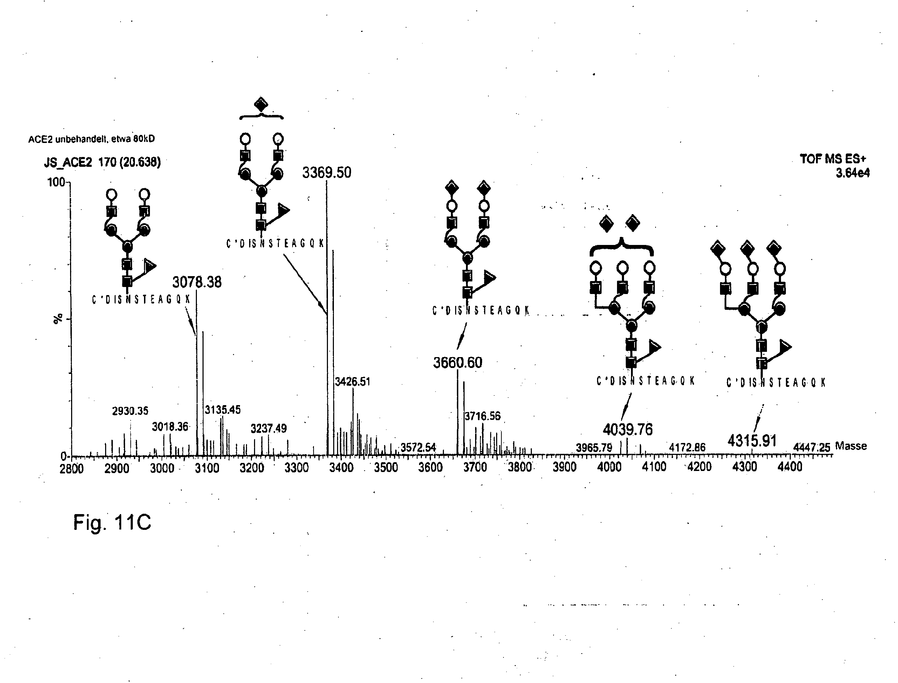

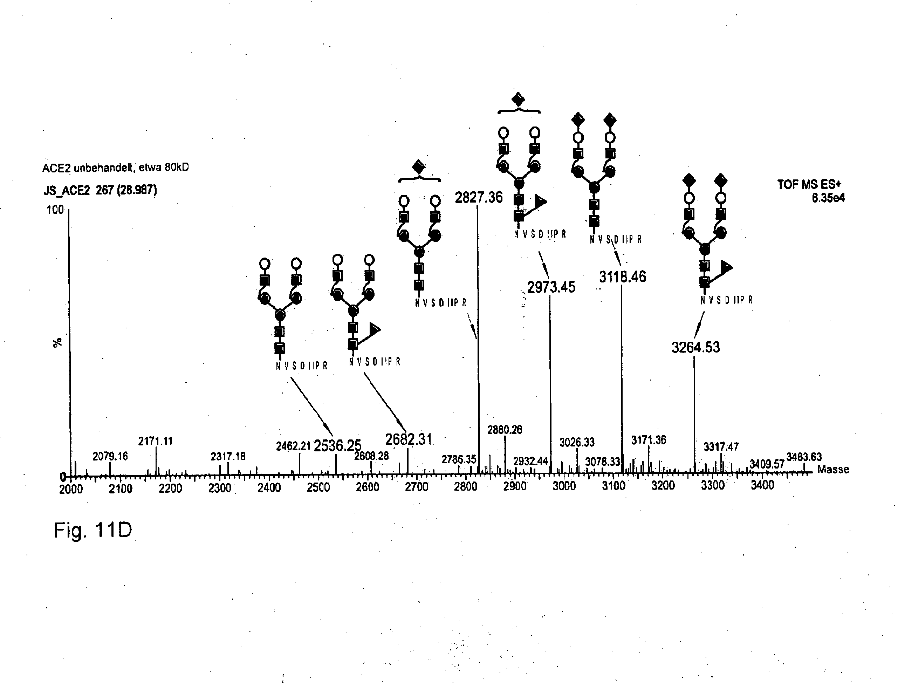

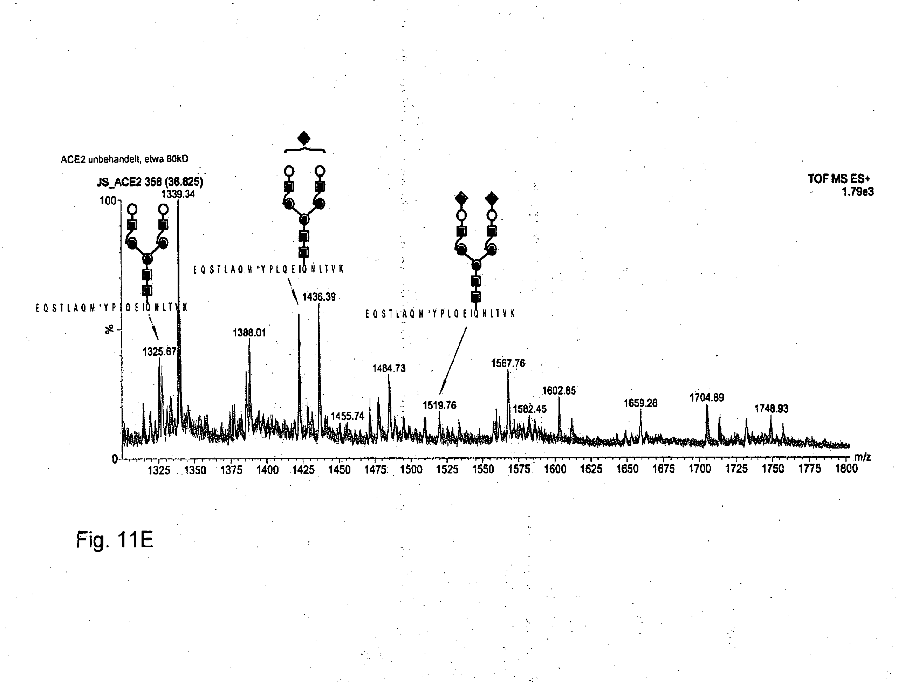

Die Struktur dieser Zuckerreste wurde durch carbon-LC-ESI-MS nach deren Abspaltung und Reduktion bestimmt. Sämtliche di-antennären Stukturen enthielten jeweils zwei α2,3-gebundene Sialinsäuren und eine α1,6-gebundene Fukose. Geringe Mengen triantennärer Strukturen wurden ebenfalls gefunden (Fig. 11). Die angewendete Selektionstrategie ermöglichte es, zu einem vollständig glykosylierten und sialinsäurehältigen Expressionsprodukt zu kommen.

### Methodik:

### Probenvorbereitung

ACE2 wurde mittels SDS-PAGE aufgetrennt, entfärbt, alkyliert und mittels Trypsin verdaut. (Kolarich et al., Proteomics 6 (2006): 3369-3380). Gelextrakte wurden getrocknet und vor der LC-MS Analytik in 0,1% Ameisensäure gelöst. Für die Zuckeranalytik wurden die Peptide mittels PNGase-F verdaut und über C18 SPE Säulchen gereinigt

### MS-Analytik

Alle Massenspektroskopischen Analysen wurden mittels Q-TOF Ultima Global (Waters Micromass) mit einer Standard-electrospray Einheit und einem Cap-LC system (Waters Micromass) durchgeführt (Kolarich 2006).

Die Proben wurden auf einer Aquasil C18 Vorsäule (30 x 0.32 mm, Thermo Electron) in Wasser angereichert. Als Trennsäule wurde eine C18 Säule (100 x 0.18 mm, Thermo Electron) unter Verwendung eines Acetonitrilgradienten eingesetzt. Die Proben wurden im MS und im MSMS Modus vermessen.

### Analytik der freien N-Glykane

Mittels Borohydrid reduzierte Glykane wurden auf einer porösen Graphit Säule getrennt und über ihre Masse charakterisiert.

### Beispiel 7: Dimerisierung von rhACE2

Nach Beispiel 1 hergestellte ACE2 wird als Dimer erhalten und in diesem Beispiel als solches analysiert, ohne die Dimere zu trennen. Durch native Behandlung bleibt das Dimer erhalten im Gegensatz zu denaturierenden Analysen (Fig. 3). Bedingt durch die Dimerisierung von ACE2 sind sämtliche hydrophobe Proteineinheiten in das Innere des Komplexes gerichtet, wobei die geladenen Reste, wie N-gebundenen Zuckerketten nach außen ragen und die Struktur im ebenfalls geladenen physiologischen Milieu solvatisieren. Diese Dimerisierung durch Expression eines vollständig N-Glykosylierten ACE2 wurde in Gegenwart von Zn²⁺ festgestellt. Der Dimer-Komplex besteht hierbei aus 2 identischen Untereinheiten, die elektrostatisch aneinander gebunden sind und sich auch nicht mehr in physiologischen Lösungen trennen. Es kommt hierbei zur Sekretion eines Glykoproteins mit jeweils 14 stark geladenen Sialinsäurestrukturen auf jedem ACE2 Molekül sowie 28 Sialinsäurestrukturen im Dimer. Jeweils 2 Zn²⁺ Atome werden in den Komplex eingebaut und stabilisieren dessen Struktur. Die starke Ladung der Zuckerketten solvatisiert das Molekül in physiologischen wäßrigen Lösungen und zwingt die zugehörigen geladenen Proteindomänen nach außen. Der Produktionsprozeß wurde so aufgebaut, dass im Endprodukt ausschließlich ACE2 Dimere vorkommen.

Dies wird dadurch ermöglicht, dass bei Generierung des rACE2 genügend Zn²⁺-Ionen vorhanden sind (vorzugsweise kommen 1,5-5 micromolar Zn²⁺ zur Anwendung, insbesondere kann die Fermentation bei 2,5-3,5 uM Zn²⁺ durchgeführt werden) und anschließend die weiteren Behandlungsschritte in Anwesenheit von Zn²⁺-Ionen vorgenommen werden.

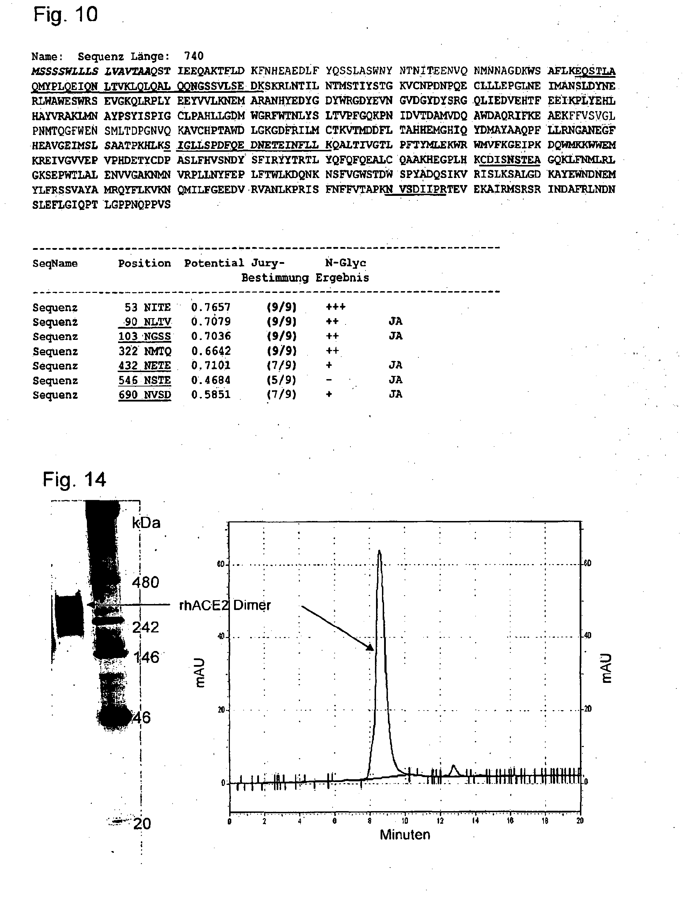

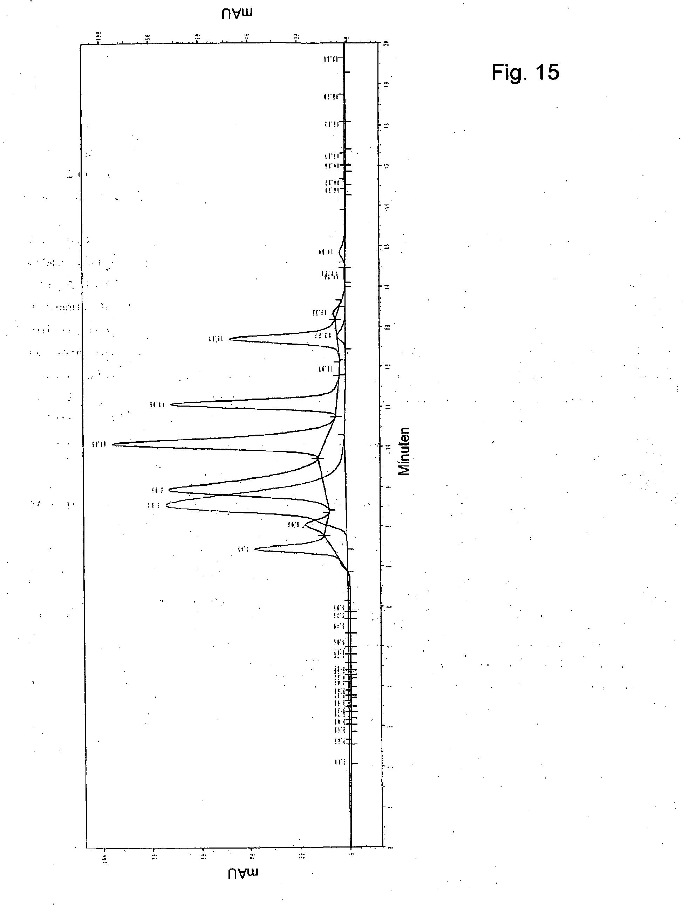

In Figur 14 und 15 wurde die Dimerisierung des ACE2 Komplexes mit unterschiedlichen Methoden nachgewiesen. In der nativen Poly-Acrylamid-Gel-Elektrophorese wurde das Protein nach Silberfärbung als einzelne Bande einer Größe von ca. 250 kDa nachgewiesen. Mittels Größentrennungschromatographie auf einer Zorbax G-450 Säule in Gegenwart von 10% Acetonitril in 220 mM Na-Phosphatpuffer bei pH 7,0 erscheint das Produkt ebenfalls als einzelner Peak bei einer einem Molekulargewicht von ca. 250 kDa entsprechenden Retentionszeit. Bemerkenswert ist in beiden Fällen, dass ausschließlich dimerisiertes Protein nachgewiesen werden kann. Weder Monomerstrukturen, noch höhermolekulare Aggregate konnten nachgewiesen werden.

### Beispiel 8: ACE2 Dimere - Unterschiede zu membranständigem ACE2

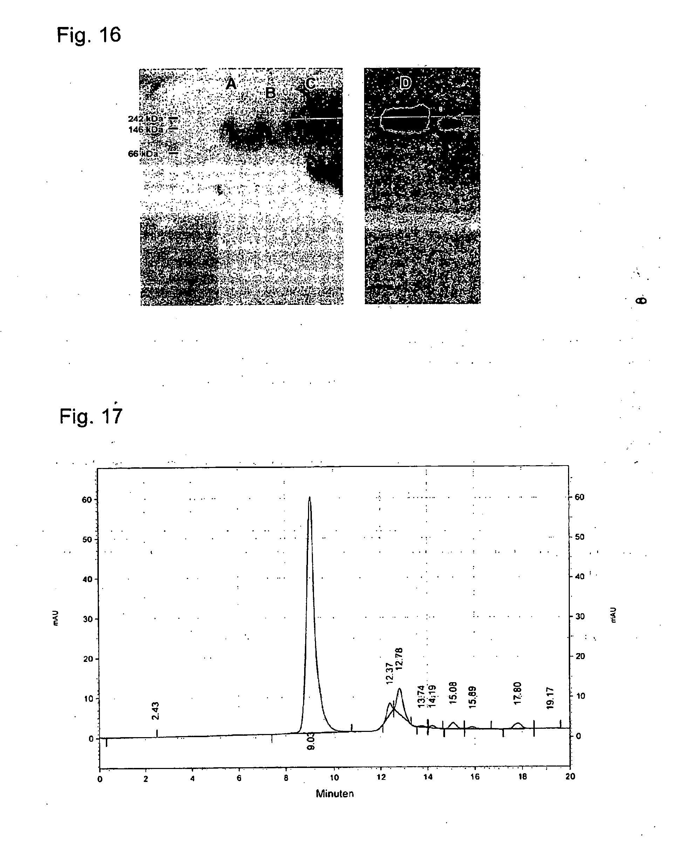

ACE2 wird als Transmembranprotein in allen höheren Spezies vorwiegend auf Nieren-, Herz-, Lungen- und Leberzellen als wesentliches Enzym des Renin Angiotensin Systems exprimiert. Membranverankertes ACE2 umgibt sich naturgemäß in der membranären Lipiddoppelschicht mit anderen Membranproteinen, welche ACE2 in einer aktiven Konformation stabilisieren und ebenfalls die extrazelluläre Domäne von ACE2 vor proteolytischem Abbau schützen. Um die pharmakologischen Eigenschaften von löslichem ACE2 und im Speziellen die Aktivität und Stabilität des löslichen Proteins zu erhöhen, wurde eine Expressions- und Produktionsstrategie gewählt, die ausschließlich zu einer stabilen ACE2 Dimerstrukturen führt. Es ist vor allem die C-terminale Domäne des Proteins und dessen post-translationale Modifikationen, welche maßgeblich für die Dimerisierung verantwortlich sind. Um die Besonderheit der ACE2 Dimerstruktur hervorzuheben, wurde die Struktur von membranständigen ACE2 mittels nativem PAGE und ACE2 spezifischem Westernblot analysiert (Fig. 16). In den Spuren A und B sind Zellextrakte (Cortex und Gehirn) sowie in Spur C ein Zellextrakt eines Produktionsklons für ACE2 Dimere, produziert nach Beispiel 1, aufgetragen. Das membranständige Produkt weist hierbei ein deutlich geringeres Molekulargewicht im Vergleich zum Expressionsprodukt nach Beispiel 1 (C und D) auf, obwohl erstes nur aus dem extrazellulären Teil besteht. Dies deutet drauf hin, daß membranständiges ACE2 als Monomer vorliegt. Das Expressionsprodukt hingegen besteht aus Dimeren, die dem Produkt pharmakologische Vorteile verleihen. In Spur D wurde das aufgereinigte finale Produkt analysiert. Dieses weist nur mehr eine einzige Bande eines Molekulargewichtes von ca. 240 kDa auf.

### Beispiel 9: verbesserten pharmakologischen Eigenschaften der ACE2 Dimere

APN 01 ist die Bezeichnung einer physiologischen ACE2 Proteinformulierung, die ausschließlich aus ACE2 Dimerstrukturen besteht. Jeweils zwei ACE2 Monomere bilden hierbei nicht-kovalent aneinander gebundene Komplexe. Die molekularbiologischen Konstrükte, die Expressionszellinie, der Fermentationsprozeß, die Aufreinigung und der Puffer für Lagerung und Applikation sind so selektioniert bzw. konzipiert worden, dass im finalen Produkt ausschließlich stabile ACE2-Dimere vorkommen. Das Dimer aggregiert weder zu größeren Komplexen noch dissoziiert es unter physiologischen Bedingungen zu Monomeren.

In Fig. 15 (Analytisches SEC-HPLC Chromatogram: APN 01 im Vergleich zu einem Größentrennungsstandard (Bio-RAD GF-Stahdard, blaue Kurve). Laufbedingungen: Säule: Zorbax GF250, Puffer: 220 mM Na₂HPO₄ + 10% CH₃CN , pH 8.0 bei 1 ml/min), eluiert APN 01 in Form eines einzigen Peaks bei 8,6 Minuten, der sich bezüglich seiner Retentionszeit zwischen Thyroglobulin (680 kDa, Retentionszeit 7,4 Minuten) und bovinem Gamma-globulin (158 kDa, Restentionszeit 8,9 Minuten) befindet. Die berechnete Molekularmasse dieses Komplexes beträgt ca. 214 kDa. Dies entspricht in etwa dem erwarteten Molekulargewicht von zwei ACE2 Einheiten von jeweils 102 kDa.

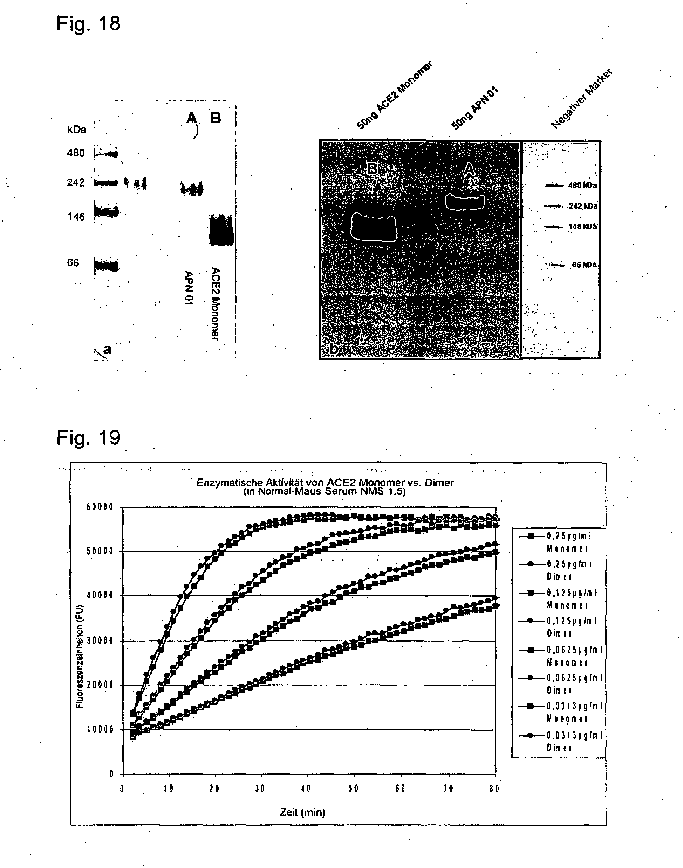

Zu Vergleichszwecken wurde als Referenzmaterial ACE2 Monomere in CHO-Zellen hergestellt, welche ebenfalls mittels SEC-HPLC analysiert wurden. Das diesbezügliche Chromatogramm ist in Fig. 17 dargestellt. Die Monomerform eluiert bei einer Retentionszeit von 9,0 Minuten, was einem Molekulargewicht um 110 kDa entspricht. Beide Produkte wurden ferner mittels PAGE verglichen (siehe Fig. 18a). Während APN 01 (A) eine Proteinbande mit einem Molekulargewicht von ca. 240 kDa aufweist, erscheint die Monomerform (B) bei ca. 100 kDa. Mittels ACE2 spezifischem Westernblot wurde die Identität beider Produkte bestätigt, wie in Fig. 18b ersichtlich ist.

Beide Produkte zeigen eine identische spezifische Enzymaktivität, gemessen anhand eines Aktivitätstests unter Verwendung eines fluoreszenzmarkierten Substrates. Wie in Fig. 19 ersichtlich ist, liegen die Kurven derselben Enzymkonzentration der Monomer- und Dimerform übereinander.

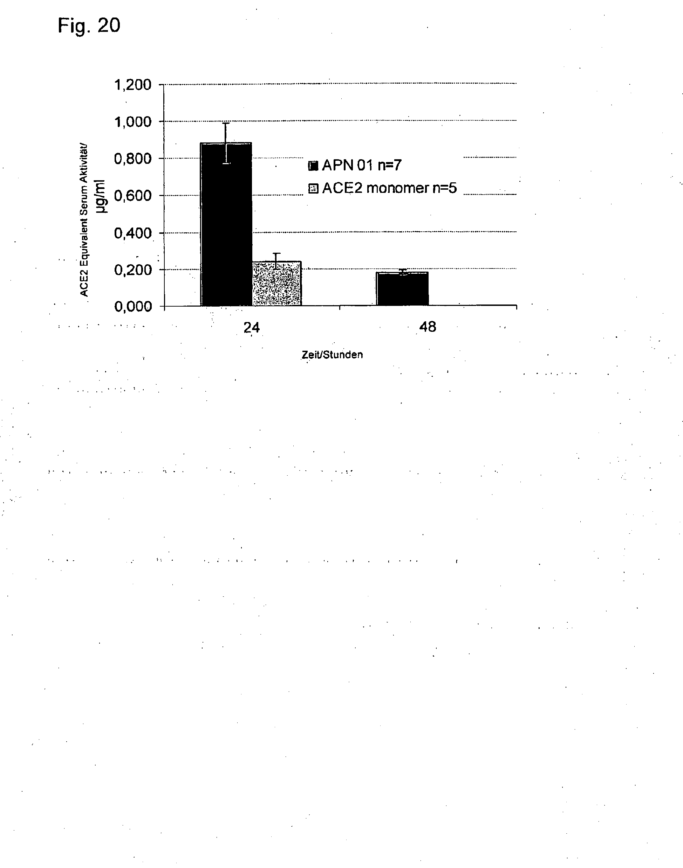

Um die pharmakologischen Eigenschaften beider Produkte zu vergleichen, wurden beide Präparationen Bl-6 Mäusen intraperitoneal als Bolusinjektion von 2,5 mg/kg verabreicht und die ACE2 Enzymaktivität in Serumproben nach 24 und 48 Stunden gemessen (siehe Fig. 20). Die Proteinkonzentration wurde so eingestellt, dass alle Tiere jeweils 100 µl einer physiologischen Proteinlösung erhielten. Jeweils 7 Tiere wurden mit APN 01 (ACE2-Dimer) und 5 Tiere mit ACE2 Monomer behandelt. Während vor ACE2 Gabe in keiner Serumprobe ACE2 Aktivität meßbar war, konnte nach der Verabreichung in allen Fällen systemisch ACE2 Aktivität gemessen werden. Bereits 24 Stunden nach Applikation unterschieden sich beide Gruppen signifikant (ttest, p<0,001): Während in den Tieren, die mit dem ACE2-Dimer behandelt wurden, eine Aktivität entsprechend einer ACE2 Konzentration von 0,9 µg/ml gemessen wurde, konnte in der Gruppe, welche ACE2 Monomer erhielt, nur die einer Konzentration von 0,2 µg/ml entsprechende Aktivität gefunden werden. Nach 48 Stunden fanden sich in der Gruppe, die ACE2 Homodimere erhielt, noch 0,2 µg/ml Aktivität, während in der Gruppe, die das Monomer erhielt, keine systemische ACE2 Aktivität mehr nachgewiesen werden konnte. Diese Daten zeigen einen sehr deutlichen pharmakologischen Vorteil der ACE2 Dimerform.

## Patentansprüche

1. Präparation umfassend rekombinante, glycosylierte, lösliche, menschliche, dimere, enzymatisch aktive ACE2 Polypeptide, wobei die ACE2 Dimere jeweils zwei ACE2 Monomereinheiten umfassen, welche nicht-kovalent verbunden sind, und wobei der Anteil der ACE2 Dimere an den gesamten ACE2 Molekülen mindestens 80% ist.

2. Präparation nach Anspruch 1, wobei der Anteil der ACE2 Dimere mindestens 90% ist.

3. Präparation nach Anspruch 2, wobei der Anteil der ACE2 Dimere mindestens 95% ist.

4. Präparation nach einem beliebigen der Ansprüche 1 bis 3, wobei der Anteil der ACE2 Polypeptide mit Transmembrandomänen unter 20% der gesamten ACE2 Polypeptide ist.

5. Präparation nach Anspruch 4, wobei der Anteil der ACE2 Polypeptide mit Transmembrandomänen unter 5% ist.

6. Präparation nach Anspruch 5, wobei der Anteil der ACE2 Polypeptide mit Transmembrandomänen unter 1% ist.

7. Präparation nach Anspruch 6, wobei der Anteil der ACE2 Prolypeptide mit Transmembrandomänen 0% ist.

8. Präparation nach einem beliebigen der Ansprüche 1 bis 7, wobei der Anteil an ACE2 Multimeren unter 20% der gesamten ACE2 Polypeptide ist.

9. Präparation nach Anspruch 8, wobei der Anteil an ACE2 Multimeren unter 5% ist.

10. Präparation nach Anspruch 9, wobei der Anteil an ACE2 Multimeren unter 1% ist.

11. Präparation nach einem beliebigen der Ansprüche 1 bis 10, wobei der Anteil an ACE2 Monomeren unter 20% der gesamten ACE2 Polypeptide ist.

12. Präparation nach Anspruch 11, wobei der Anteil an ACE2 Multimeren unter 10% ist.

13. Präparation nach Anspruch 12, wobei der Anteil an ACE2 Multimeren unter 5% ist.

14. Präparation nach Anspruch 13, wobei der Anteil an ACE2 Multimeren unter 1% ist.

15. Präparation nach einem beliebigen der Ansprüch 1 bis 14, wobei die ACE2 Dimere als Homodimere vorliegen.

16. Präparation nach einem beliebigen der Ansprüche 1 bis 15, wobei die ACE2 Dimere jeweils zwei Zink-Ionen enthalten.

17. Präparation nach einem beliebigen der Ansprüche 1 bis 16, wobei die Glycogruppen einer ACE2 Polypeptid Monomereinheit in Summe mindestens 10 Sialylsäure-Reste aufweisen.

18. Präparation nach einem beliebigen der Ansprüche 1 bis 17, wobei die ACE2 Polypeptide einen Zuckeranteil von größer als 10% (Massen-%, bezogen auf das gesamte ACE2-Molekül) aufweisen.

19. Präparation nach Anspruch 18, wobei die ACE2 Polypeptide einen Zuckeranteil von größer als 15% aufweisen.

20. Präparation nach Anspruch 19, wobei die ACE2 Polypeptide einen Zuckeranteil von größer als 20% aufweisen.

21. Präparation nach einem beliebigen der Ansprüche 1 bis 20, wobei die ACE2 Polypeptide aus den Aminosäuren 18-740 von SEQ ID NO: 1 oder enzymatisch aktiven Fragmenten davon bestehen.

22. Präparation nach einem beliebigen der Ansprüche 1 bis 20, wobei die ACE2 Polypeptide SEQ ID NO: 1 oder enzymatisch aktiven Fragmente davon umfassen.

23. Präparation nach einem beliebigen der Ansprüche 1 bis 22, wobei mindestens 70% der glykosylierten N-Glykosylierungspositionen eine Struktur unabhängig voneinander ausgewählt aus den Formeln 1-8: 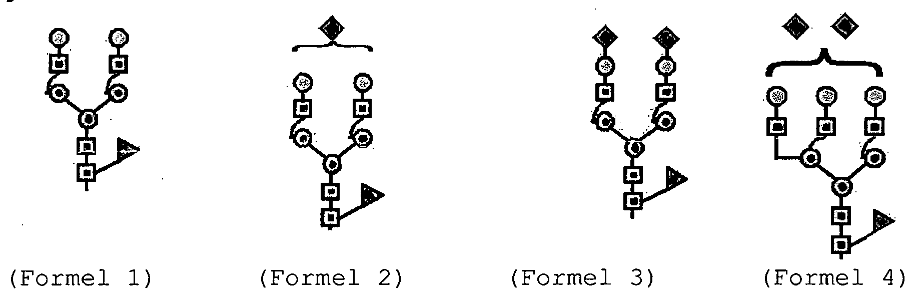 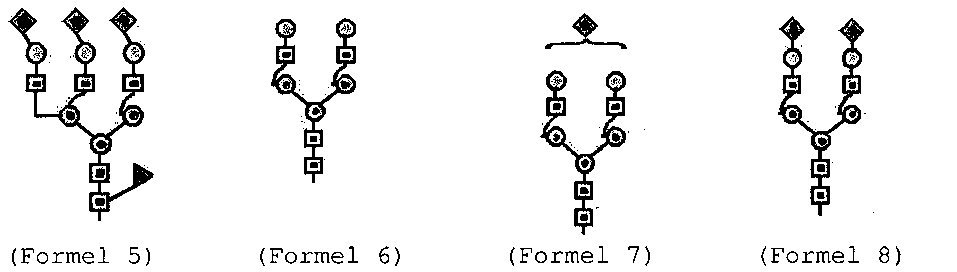 wobei
| | | | |
|---|---|---|---|
| G lc N A c | 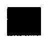 | F u c | 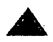 |
| M a n | 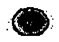 | X y l | 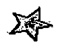 |
| G a l | 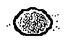 | N e u 5 A c | 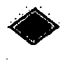 |
ist, aufweisen.

24. Präparation nach einem beliebigen der Ansprüche 1 bis 23, wobei alle der möglichen N-Glykosylierungspositionen der ACE2 Polypeptide glykosyliert sind.

25. Präparation nach einem beliebigen der Ansprüche 1 bis 24, wobei mindestens 70%, mindestens 80%, mindestens 90%, oder 100% der glykosylierten N-Glykosylierungsstellen Sialinsäure aufweisen, und optional die N-Glykosylierungsstellen entsprechend Asn53, Asn90, Asn103, Asn322, Asn432, Asn546, Asn690 der SEQ ID NO: 1 sialysiert sind.

26. Präparation nach einem beliebigen der Ansprüche 1 bis 25, wobei mindestens 30%, mindestens 40%, mindestens 55%, oder mindestens 70% der glykosylierten N-Glykosylierungsstellen mindestens zwei Sialinsäuren aufweisen.

27. Präparation nach einem beliebigen der Ansprüche 1 bis 26, wobei der Anteil an ACE Polypeptiden mit einem Molekulargewicht bestimmt durch Gelelektrophorese von weniger als 100 kDa weniger als 20% ist.

28. Präparation nach einem beliebigen der Ansprüche 1 bis 27, **dadurch gekennzeichnet, dass** der Anteil an ACE2 Polypeptiden mit N-glykosyliertem Asparagin ausgewählt aus der Gruppe entsprechend Asn53, Asn90, Asn90, Asn322, Asn432, Asn546 und Asn690 der SEQ ID NO: 1 größer als 60%, größer als 70%, größer als 80%, größer als 90%, größer als 99%, oder 100%, ist und optional mit einem Glykan der Strukturen nach Formel 1, 2, 3, 4, 5, 6, 7 oder 8 wie in Anspruch 23 definiert.

29. Präparation nach einem beliebigen der Ansprüche 1 bis 28, **dadurch gekennzeichnet, dass** der Anteil an ACE2 Polypeptiden mit O-glykosyliertem Serin entsprechend Ser740 der SEQ ID NO: 1 größer als 60%, größer als 70%, größer als 80%, größer als 90%, größer als 99%, oder 100%, ist.

30. Präparation nach einem beliebigen der Ansprüche 1 bis 29 mit einer katalytischen Aktivität kkat von mindestens 4 s⁻¹, vorzugsweise von mindestens 5 s⁻¹, insbesondere bevorzugt von mindestens 6 s⁻¹, speziell bevorzugt von mindestens 7 s⁻¹, am meisten bevorzugt von mindestens 7,6 s⁻¹, bezogen auf den Umsatz von AngII (Angiotensin II) zu Ang 1-7 (Angiotensin 1-7).

31. Medikament umfassend eine Präparation wie in einem beliebigen der Ansprüche 1 bis 30 definiert.

32. Präparation nach einem beliebigen der Ansprüche 1 bis 29 zur Anwendung in einer Therapie.

## Claims

1. Preparation comprising recombinant, glycosylated, soluble, human, dimeric, enzymatically active ACE2 polypeptides, wherein the ACE2 dimers each comprise two ACE2 monomer units which are non-covalently bound, and wherein the fraction of ACE2 dimers of the total ACE2 polypeptides is at least 80%.

2. A preparation according to claim 1, wherein the fraction of ACE2 dimers is at least 90 %.

3. A preparation according to claim 2, wherein the fraction of ACE2 dimers is at least 95 %.

4. A preparation according to any one of claims 1 to 3, wherein the fraction of ACE2 polypeptides with transmembrane domains is less than 20 % of the total ACE2 polypeptides.

5. A preparation according to claim 4, wherein the fraction of ACE2 polypeptides with transmembrane domains is less than 5 %.

6. A preparation according to claim 5, wherein the fraction of ACE2 polypeptides with transmembrane domains is less than 1 %

7. A preparation according to claim 6, wherein the fraction of ACE2 polypeptides with transmembrane domains is 0 %.

8. A preparation according to any one of claims 1 to 7, wherein the fraction of ACE2 multimers is less than 20 % of the total ACE2 polypeptides.

9. A preparation according to claim 8, wherein the fraction of ACE2 multimers is less than 5%.

10. A preparation according to claim 9, wherein the fraction of ACE2 multimers is less than 1 %.

11. A preparation according to any one of claims 1 to 10, wherein the fraction of ACE2 monomers is less than 20% of the total ACE2 polypeptides.

12. A preparation according to claim 11, wherein the fraction of ACE2 monomers is less than 10%.

13. A preparation according to claim 12, wherein the fraction of ACE2 monomers is less than 5%.

14. A preparation according to claim 13, wherein the fraction of ACE2 monomers is less than 1%.

15. A preparation according to any one of claims 1 to 14, wherein the ACE2 dimers are present as homodimers.

16. A preparation according to any one of claims 1 to 15, wherein the ACE2 dimers each contain two zinc ions.

17. A preparation according to any one of claims 1 to 16 wherein the glycosyl groups of the ACE2 polypeptide monomers have a total of at least 10 sialic acid residues.

18. A preparation according to any one of claims 1 to 17 wherein the ACE2 polypeptides have a proportion of sugar of more than 10 % (weight % with respect to the total ACE2 polypeptides).

19. A preparation according to claim 18 wherein the ACE2 polypeptides have a proportion of sugar of more than 15 %.

20. A preparation according to claim 19 wherein the ACE2 polypeptides have a proportion of sugar of more than 20 %.

21. A preparation according to any one of claims 1 to 20, wherein the ACE2 polypeptides consist of amino acids 18 - 740 of SEQ ID NO: 1 or enzymatically active fragments thereof.

22. A preparation according to any one of claims 1 to 20, wherein the ACE2 polypeptides comprises SEQ ID NO: 1 or enzymatically active fragments thereof.

23. A preparation according to any one of claims 1 to 22, wherein at least 70 % of the glycosylated N-glycosylation sites of the ACE2 polypeptides have a structure, independently of each other, selected from formulae 1 to 8: 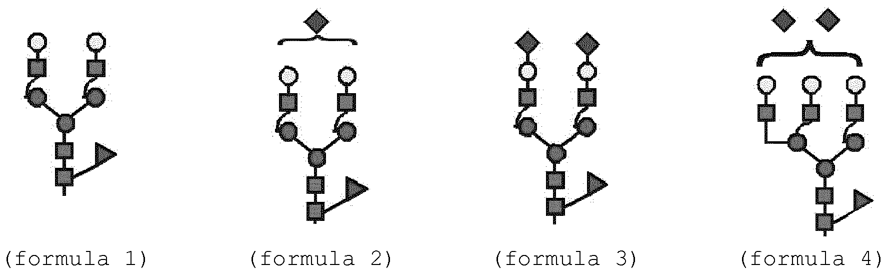 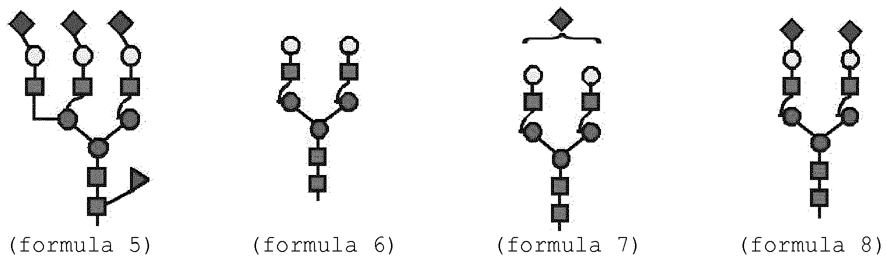 wherein
| | | | |
|---|---|---|---|
| G lc N A c | 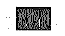 | F u c | 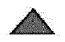 |
| M a n | 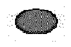 | X y l | 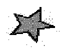 |
| G a l | 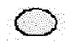 | N e u 5 A c | 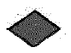 |

24. preparation according to any one of claims 1 to 23, wherein all of the possible N-glycosylation sites of the ACE2 polypeptides are glycosylated.

25. A preparation according to any one of claims 1 to 24, wherein at least 70 %, at least 80 %, at least 90 % or at least 100 % of the glycosylated N-glycosylation sites of the ACE2 polypeptides have sialic acids, and optionally the N-glycosylation sites corresponding to Asn53, Asn90, Asn103, Asn322, Asn432, Asn546, Asn690 of SEQ 1 D NO: 1 are sialyzed.

26. A preparation according to any one of claims 1 to 25, wherein at least 30 %, at least 40 %, at least 55 %, or at least 70 % of the glycosylated N-glycosylation sites of the ACE2 polypeptides have at least two sialic acids.

27. Preparation according to any one of claims 1 to 26, wherein the fraction of ACE2 polypeptides with a molecular weight determined by gel electrophoresis of less than 100 kDa is less than 20 %.

28. The preparation according to any one of claims 1 to 27, **characterized in that** the fraction of ACE2 polypeptides with N-glycosylated asparagine selected from the group corresponding to Asn53, Asn90, Asn90, Asn322, Asn432, Asn546 and Asn690 of SEQ ID NO: 1 is more than 60 %, more than 70 %, more than 80 %, more than 90 %, more than 99 %, or 100 %, and optionally with a glycan with a structure in accordance with formula 1, 2, 3, 4, 5, 6, 7 or 8 as defined in claim 23.

29. A preparation according to any one of claims 1 to 28, **characterized in that** the fraction of ACE2 polypeptides with O-glycosylated serine corresponding to Ser740 of SEQ ID NO: 1 is more than 60 %, more than 70 %, more than 80 %, more than 90 %, more than 99 %, or 100 %.

30. A preparation according to any one of claims 1 to 29, with a catalytic activity, ccat, of at least 4 s⁻¹, preferably at least 5 s⁻¹, particularly preferably at least 6 s⁻¹, especially preferably at least 7 s⁻¹, most preferably at least 7.6 s⁻¹, with respect to the conversion of Ang II (Angiotensin II) to Ang 1-7 (Angiotensin 1-7).

31. A pharmaceutical composition comprising a preparation as defined in any one of claims 1 to 30.

32. A preparation according to any one of claims 1 to 29 for use in therapy.

## Revendications

1. Préparation comprenant des polypeptides ACE2 recombinants, glycosylés, solubles, humains, dimériques, enzymatiquement actifs, dans laquelle les dimères ACE2 comportent respectivement deux motifs monomériques ACE2, qui sont liés de manière non covalente, et la proportion des dimères ACE2 sur la totalité des molécules ACE2 est au moins de 80 %.

2. Préparation selon la revendication 1, dans laquelle la proportion des dimères ACE2 est au moins de 90 %.

3. Préparation selon la revendication 2, dans laquelle la proportion des dimères ACE2 est au moins de 95 %.

4. Préparation selon l'une quelconque des revendications 1 à 3, dans laquelle la proportion de polypeptides ACE2 ayant des domaines transmembranaires est inférieure à 20 % de la totalité des polypeptides ACE2.

5. Préparation selon la revendication 4, dans laquelle la proportion de polypeptides ACE2 ayant des domaines transmembranaires est inférieure à 5 %.

6. Préparation selon la revendication 5, dans laquelle la proportion de polypeptides ACE2 ayant des domaines transmembranaires est inférieure à 1 %.

7. Préparation selon la revendication 6, dans laquelle la proportion de polypeptides ACE2 ayant des domaines transmembranaires est de 0 %.

8. Préparation selon l'une quelconque des revendications 1 à 7, dans laquelle la proportion de multimères ACE2 est inférieure à 20 % de la totalité des polypeptides ACE2.

9. Préparation selon la revendication 8, dans laquelle la proportion de multimères ACE2 est inférieure à 5 %.

10. Préparation selon la revendication 9, dans laquelle la proportion de multimères ACE2 est inférieure à 1 %.

11. Préparation selon l'une quelconque des revendications 1 à 10, dans laquelle la proportion de monomères ACE2 est inférieure à 20 % de la totalité des polypeptides ACE2.

12. Préparation selon la revendication 11, dans laquelle la proportion de multimères ACE2 est inférieure à 10 %.

13. Préparation selon la revendication 12, dans laquelle la proportion de multimères ACE2 est inférieure à 5 %.

14. Préparation selon la revendication 13, dans laquelle la proportion de multimères ACE2 est inférieure à 1 %.

15. Préparation selon l'une quelconque des revendications 1 à 14, dans laquelle les dimères ACE2 se présentent sous forme d'homodimères.

16. Préparation selon l'une quelconque des revendications 1 à 15, dans laquelle les dimères ACE2 comprennent respectivement deux ions zinc.

17. Préparation selon l'une quelconque des revendications 1 à 16, dans laquelle les groupes glyco d'un motif monomérique de polypeptide ACE2 présentent au total au moins 10 résidus d'acide sialylique.

18. Préparation selon l'une quelconque des revendications 1 à 17, dans laquelle les polypeptides ACE2 présentent une proportion de sucre supérieure à 10 % (% en poids, par rapport à la molécule ACE2 totale).

19. Préparation selon la revendication 18, dans laquelle les polypeptides ACE2 présentent une proportion de sucre supérieure à 15 %.

20. Préparation selon la revendication 19, dans laquelle les polypeptides ACE2 présentent une proportion de sucre supérieure à 20 %.

21. Préparation selon l'une quelconque des revendications 1 à 20, dans laquelle les polypeptides ACE2 se composent des acides aminés 18 à 740 de la SEQ ID NO : 1 ou de fragments enzymatiquement actifs de celle-ci.

22. Préparation selon l'une quelconque des revendications 1 à 20, dans laquelle les polypeptides ACE2 comprennent la SEQ ID NO : 1 ou des fragments enzymatiquement actifs de celle-ci.

23. Préparation selon l'une quelconque des revendications 1 à 22, dans laquelle au moins 70 % des positions de N-glycosylation glycosylées présentent une structure choisie indépendamment les unes des autres parmi les formules 1 à 8 : 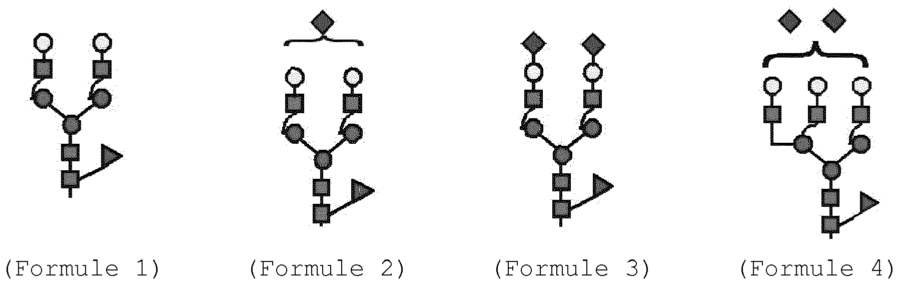 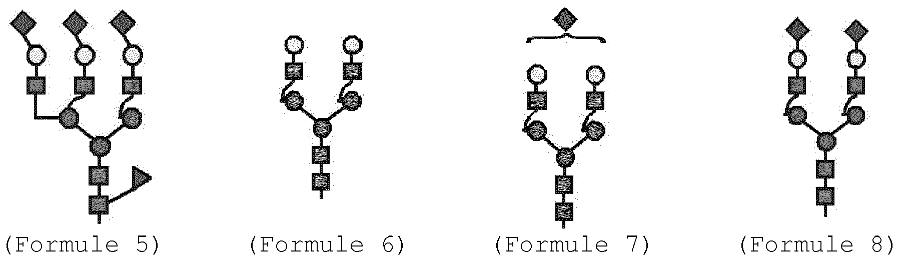 dans lesquelles on a
| | | | |
|---|---|---|---|
| G lc N A c | 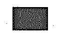 | F u c | 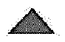 |
| M a n | 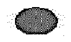 | X y l | 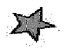 |
| G a l | 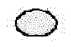 | N e u 5 A c | 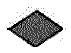 |

24. Préparation selon l'une quelconque des revendications 1 à 23, dans laquelle toutes les positions de N-glycosylation possibles des polypeptides ACE2 sont glycosylées.

25. Préparation selon l'une quelconque des revendications 1 à 24, dans laquelle au moins 70 %, au moins 80 %, au moins 90 %, ou 100 % des sites de N-glycosylation glycosylés présentent de l'acide sialique et les sites de N-glycosylation correspondant à Asn53, Asn90, Asn103, Asn322, Asn432, Asn546, Asn690 de la SEQ ID NO : 1 sont éventuellement sialysés.

26. Préparation selon l'une quelconque des revendications 1 à 25, dans laquelle au moins 30 %, au moins 40 %, au moins 55 %, ou au moins 70 % des sites de N-glycosylation glycosylés présentent au moins deux acides sialiques.

27. Préparation selon l'une quelconque des revendications 1 à 26, dans laquelle la proportion des polypeptides ACE ayant un poids moléculaire déterminé par électrophorèse sur gel inférieur à 100 kDa est inférieure à 20 %.

28. Préparation selon l'une quelconque des revendications 1 à 27, **caractérisée en ce que** la proportion de polypeptides ACE2 ayant de l'asparagine N-glycosylée choisie dans le groupe correspondant à Asn53, Asn90, Asn322, Asn432, Asn546 et Asn690 de la SEQ ID NO : 1 est supérieure à 60 %, supérieure à 70 %, supérieure à 80 %, supérieure à 90 %, supérieure à 99 %, ou est de 100 % et a éventuellement un glycane des structures répondant aux formules 1, 2, 3, 4, 5, 6, 7 ou 8, telles que définies dans la revendication 23.

29. Préparation selon l'une quelconque des revendications 1 à 28, **caractérisée en ce que** la proportion de polypeptides ACE2 ayant une sérine O-glycosylée correspondant à Ser740 de la SEQ ID NO : 1 est supérieure à 60 %, supérieure à 70 %, supérieure à 80 %, supérieure à 90 %, supérieure à 99 %, ou est de 100 %.

30. Préparation selon l'une quelconque des revendications 1 à 29 ayant une activité catalytique kkat d'au moins 4 s⁻¹, de préférence d'au moins 5 s⁻¹, particulièrement préférentiellement d'au moins 6 s⁻¹, spécifiquement préférentiellement d'au moins 7 s⁻¹, de manière la mieux préférée d'au moins 7,6 s⁻¹, par rapport au volume de AngII (angiotensine II) sur Ang 1-7 (angiotensine 1-7).

31. Médicament comprenant une préparation telle que définie dans l'une quelconque des revendications 1 à 30.

32. Préparation selon 1"une quelconque des revendications 1 à 29 pour l'utilisation dans une thérapie.


### SEQUENCE LISTING

<110> Apeiron Biologics Forschungs- und Entwicklungs GmbH
<120> ACE2 Polypeptid
<130> r52100
<150> AT A 913/2007
   <151> 2007-06-12
<150> EP 08450052.9
   <151> 2008-04-08
<160> 1
<170> PatentIn version 3.5
<210> 1
   <211> 740
   <212> PRT
   <213> homo sapiens
<400> 1
   Met Ser Ser Ser Ser Trp Leu Leu Leu Ser Leu Val Ala Val Thr Ala
   1               5                   10                  15
   Ala Gln Ser Thr Ile Glu Glu Gln Ala Lys Thr Phe Leu Asp Lys Phe
               20                  25                  30
   Asn His Glu Ala Glu Asp Leu Phe Tyr Gln Ser Ser Leu Ala Ser Trp
           35                  40                  45
   Asn Tyr Asn Thr Asn Ile Thr Glu Glu Asn Val Gln Asn Met Asn Asn
       50                  55                  60
   Ala Gly Asp Lys Trp Ser Ala Phe Leu Lys Glu Gln Ser Thr Leu Ala
   65                  70                  75                  80
   Gln Met Tyr Pro Leu Gln Glu Ile Gln Asn Leu Thr Val Lys Leu Gln
                   85                  90                  95
   Leu Gln Ala Leu Gln Gln Asn Gly Ser Ser Val Leu Ser Glu Asp Lys
               100                 105                 110
   Ser Lys Arg Leu Asn Thr Ile Leu Asn Thr Met Ser Thr Ile Tyr Ser
           115                 120                 125
   Thr Gly Lys Val Cys Asn Pro Asp Asn Pro Gln Glu Cys Leu Leu Leu
       130                 135                 140
   Glu Pro Gly Leu Asn Glu Ile Met Ala Asn Ser Leu Asp Tyr Asn Glu
   145                 150                 155                 160
   Arg Leu Trp Ala Trp Glu Ser Trp Arg Ser Glu Val Gly Lys Gln Leu
                   165                 170                 175
   Arg Pro Leu Tyr Glu Glu Tyr Val Val Leu Lys Asn Glu Met Ala Arg
               180                 185                 190
   Ala Asn His Tyr Glu Asp Tyr Gly Asp Tyr Trp Arg Gly Asp Tyr Glu
           195                 200                 205
   Val Asn Gly Val Asp Gly Tyr Asp Tyr Ser Arg Gly Gln Leu Ile Glu
       210                 215                 220
   Asp Val Glu His Thr Phe Glu Glu Ile Lys Pro Leu Tyr Glu His Leu
   225                 230                 235                 240
   His Ala Tyr Val Arg Ala Lys Leu Met Asn Ala Tyr Pro Ser Tyr Ile
                   245                 250                 255
   Ser Pro Ile Gly Cys Leu Pro Ala His Leu Leu Gly Asp Met Trp Gly
               260                 265                 270
   Arg Phe Trp Thr Asn Leu Tyr Ser Leu Thr Val Pro Phe Gly Gln Lys
           275                 280                 285
   Pro Asn Ile Asp Val Thr Asp Ala Met Val Asp Gln Ala Trp Asp Ala
       290                 295                 300
   Gln Arg Ile Phe Lys Glu Ala Glu Lys Phe Phe Val Ser Val Gly Leu
   305                 310                 315                 320
   Pro Asn Met Thr Gln Gly Phe Trp Glu Asn Ser Met Leu Thr Asp Pro
                   325                 330                 335
   Gly Asn Val Gln Lys Ala Val Cys His Pro Thr Ala Trp Asp Leu Gly
               340                 345                 350
   Lys Gly Asp Phe Arg Ile Leu Met Cys Thr Lys Val Thr Met Asp Asp
           355                 360                 365
   Phe Leu Thr Ala His His Glu Met Gly His Ile Gln Tyr Asp Met Ala
       370                 375                 380
   Tyr Ala Ala Gln Pro Phe Leu Leu Arg Asn Gly Ala Asn Glu Gly Phe
   385                 390                 395                 400
   His Glu Ala Val Gly Glu Ile Met Ser Leu Ser Ala Ala Thr Pro Lys
                   405                 410                 415
   His Leu Lys Ser Ile Gly Leu Leu Ser Pro Asp Phe Gln Glu Asp Asn
               420                 425                 430
   Glu Thr Glu Ile Asn Phe Leu Leu Lys Gln Ala Leu Thr Ile Val Gly
           435                 440                 445
   Thr Leu Pro Phe Thr Tyr Met Leu Glu Lys Trp Arg Trp Met Val Phe
       450                 455                 460
   Lys Gly Glu Ile Pro Lys Asp Gln Trp Met Lys Lys Trp Trp Glu Met
   465                 470                 475                 480
   Lys Arg Glu Ile Val Gly Val Val Glu Pro Val Pro His Asp Glu Thr
                   485                 490                 495
   Tyr Cys Asp Pro Ala Ser Leu Phe His Val Ser Asn Asp Tyr Ser Phe
               500                 505                 510
   Ile Arg Tyr Tyr Thr Arg Thr Leu Tyr Gln Phe Gln Phe Gln Glu Ala
               515                 520                 525
   Leu Cys Gln Ala Ala Lys His Glu Gly Pro Leu His Lys Cys Asp Ile
       530                 535                 540
   Ser Asn Ser Thr Glu Ala Gly Gln Lys Leu Phe Asn Met Leu Arg Leu
   545                 550                 555                 560
   Gly Lys Ser Glu Pro Trp Thr Leu Ala Leu Glu Asn Val Val Gly Ala
                   565                 570                 575
   Lys Asn Met Asn Val Arg Pro Leu Leu Asn Tyr Phe Glu Pro Leu Phe
               580                 585                 590
   Thr Trp Leu Lys Asp Gln Asn Lys Asn Ser Phe Val Gly Trp Ser Thr
           595                 600                 605
   Asp Trp Ser Pro Tyr Ala Asp Gln Ser Ile Lys Val Arg Ile Ser Leu
       610                 615                 620
   Lys Ser Ala Leu Gly Asp Lys Ala Tyr Glu Trp Asn Asp Asn Glu Met
   625                 630                 635                 640
   Tyr Leu Phe Arg Ser Ser Val Ala Tyr Ala Met Arg Gln Tyr Phe Leu
                   645                 650                 655
   Lys Val Lys Asn Gln Met Ile Leu Phe Gly Glu Glu Asp Val Arg Val
               660                 665                 670
   Ala Asn Leu Lys Pro Arg Ile Ser Phe Asn Phe Phe Val Thr Ala Pro
           675                 680                 685
   Lys Asn Val Ser Asp Ile Ile Pro Arg Thr Glu Val Glu Lys Ala Ile
       690                 695                 700
   Arg Met Ser Arg Ser Arg Ile Asn Asp Ala Phe Arg Leu Asn Asp Asn
   705                 710                 715                 720
   Ser Leu Glu Phe Leu Gly Ile Gln Pro Thr Leu Gly Pro Pro Asn Gln
                   725                 730                 735
   Pro Pro Val Ser
               740